# EUROPEAN PATENT SPECIFICATION

(11) **EP 3 127 478 B1**
(45) Date of publication and mention of the grant of the patent: **09.06.2021**
(21) Application number: 16189698.0
(22) Date of filing: 05.01.2012
(51) Int. Cl.: A61B 5/0295, A61B 5/02, G01N 21/25, A61B 5/024, A61B 5/00, A61B 5/1455, G01N 21/31

(54) **DEVICES AND METHODS FOR NON-INVASIVE OPTICAL PHYSIOLOGICAL MEASUREMENTS**
VORRICHTUNGEN UND VERFAHREN FÜR PHOTOPLETHYSMOGRAPHISCHE MESSUNGEN
DISPOSITIFS ET PROCÉDÉS POUR DES MESURES PHOTOPLÉTHYSMOGRAPHIQUES

(30) Priority: 20.01.2011 US 201113010705
(43) Date of publication of application: 08.02.2017
(62) Divisional of application: 12736672.2
(73) Proprietor: Nitto Denko Corporation, Ibaraki-shi, Osaka 567-8680 (JP)
(72) Inventor: THAVEEPRUNGSRIPORN, Visit, 138623 Singapore (SG); KASSIM, Md. Irwan bin Md., 138623 Singapore (SG); NAING, Nyan Myo, 138623 Singapore (SG); SAMSUDIN, Mohamad Sulhede Bin, 138623 Singapore (SG)
(74) Representative: Stöckeler, Ferdinand

(56) References cited:
- US-A1- 2007 055 163
- US-A1- 2007 106 139
- US-A1- 2009 163 787
- US-A1- 2010 222 652

## Description

### BACKGROUND

### Field of the Invention

This invention broadly relate to devices and methods for non-invasive optical physiological measurements, including the detection of a photoplethysmography (PPG) signal from a user, and more particularly to a pressure detection assembly of an optical measurement device which detects an amount of pressure applied by the user to the device.

### Description of the Related Art

Optical monitoring of physiological characteristics utilizes the detection of light transmitted through a location of a user being measured. Photoplethysmography (PPG) is an optical measurement technique used to detect blood volume changes in the microvascular bed of living tissue, typically by detecting light transmitted through the ear lobe or fingertip. As arterial pulsations enter the capillary bed, changes in the volume of the blood vessels or characteristics of the blood itself modify the optical properties of the capillary bed. The PPG signal is used to measure saturation of peripheral oxygen (SpO2), which is an estimation of the level of oxygen saturation in a fluid, such as blood. The PPG signal can also be used to measure blood pressure.

A device such as a pulse oximeter is an accepted standard in clinical practice, and provides for measuring enhanced optical pulsatile signals emitted by the changes in the volume of blood flowing through a user. The pulse oximeter generally has a pair of small light emitting diodes (LEDs) facing a photodiode, with a translucent part of the user's body, usually a fingertip or an earlobe, positioned there between. The light from the LEDs passes through the tissue and is detected by the photodiode. One LED is red, with wavelength of approximately 660 nanometers (nm), and the other is infrared, with a wavelength of approximately 905, 910 or 940 nm. Absorption at these wavelengths differs significantly between oxyhemoglobin and its deoxygenated form. Therefore, the ratio of oxyhemoglobin to deoxyhemoglobin can be calculated from the ratio of the absorption of the red and infrared light, i.e. the ratio of red light to infrared light absorption of pulsating components at the measuring site.

The basic form of PPG technology requires only a few optoelectronic components: a light source to illuminate the tissue (e.g. skin) and a photodetector to measure the small variations in light intensity associated with changes in perfusion in a catchment volume.

The majority of PPG devices currently available rely on simple thresholding, or peak detection algorithms, to find principal peaks in a detected signal. However, these methods are typically unreliable when the detected signal is less than ideal. Particular problems may be encountered when the baseline of the AC signal component becomes noisy or complex, as can occur even with mild movement artifacts.

An example of combining PPG with pressure measurements is disclosed in US2009163787 A1.

Hence, there exists a need for an optical measurement device and a method for detecting a physiological signal using an optical measurement device that seek to address at least one of the above problems.

### SUMMARY

There may be provided an optical measurement device for obtaining non-invasive physiological measurements from a portion of living tissue and method of using the same, which more particularly includes a pressure detection assembly configured to detect and display an amount of pressure applied by a body part of a user to the device during the optical measurement. When a user applies an appropriate amount of pressure to the optical measurement device, the resulting signal-to-noise ratio of the detected optical measurement signal, such as a photoplethysmography signal, can be increased, and a more accurate measurement can be obtained from the user. An optimum pressure can be determined in real-time by analyzing the detected optical measurement signal and correlating a high signal-to-noise ratio portion of the signal with a corresponding applied pressure. The user can be provided real-time feedback indicating whether the amount of pressure being applied by the user should be increased, decreased or maintained at the same level in order to continually obtain the highest quality signal. The optical measurement device can facilitate providing an optimal pressure determination customized for each individual user, thereby obtaining a resulting optimal measurement signal for each user.

The invention is defined by the attached set of claims.

In some example embodiments, there is provided an optical measurement device comprising an illumination and detection assembly configured to output light to a portion of living tissue of a user and detect transmitted or reflected light as a signal; a pressure assembly configured to detect an amount of pressure applied to the illumination and detection assembly by the portion of living tissue of the user; and a feedback unit configured to correlate the quality of the detected signal with the amount of applied pressure and provide feedback related to the correlation to the user.

The feedback may be an indication of whether the user should adjust the amount of pressure being applied to the illumination and detection assembly.

The feedback may display a range of optimal applied pressures along with the actual applied pressure being applied by the user.

The range of optimal applied pressures may correspond to a state of zero transmural pressure.

The feedback may be a request to the user to increase, decrease or maintain the applied pressure.

The feedback may be a real-time visual output of the detected signal and detected applied pressure.

The feedback unit may be a portable computer including a processor, memory and a display.

The illumination and detection assembly, pressure assembly and feedback unit may be integrated into a portable device.

The portable device may be configured with a plurality of illumination and detection assemblies and a plurality of pressure assemblies.

The illumination and detection assembly and the pressure assembly may communicate with the feedback unit over a wireless network.

The detected signal may be a photoplethysmography (PPG) signal.

In some example embodiments, a method for detecting a physiological signal using an optical measurement device comprises: illuminating a portion of living tissue of a user and detecting transmitted or reflected light as a signal using an illumination and detection assembly; detecting an amount of pressure applied by the portion of living tissue of the user to the illumination and detection assembly using a pressure detection assembly; correlating the quality of the detected signal with the amount of applied pressure; and providing feedback related to the correlation to the user using a feedback unit.

The method may include providing an indication to the user of whether the amount of pressure being applied to the illumination and detection assembly should be adjusted.

The method may include displaying a range of optimal applied pressures along with the actual applied pressure being applied by the user.

The method may include providing a range of optimal applied pressures along which corresponds to a state of zero transmural pressure.

The method may include requesting the user to increase, decrease or maintain the applied pressure.

The method may include displaying a real-time visual output of the detected signal and the detected applied pressure.

The method may include providing feedback on a display of a computer with a processor and a memory.

The illumination and detection assembly, pressure assembly and feedback unit may be integrated into a portable device.

The portable device may be configured with a plurality of illumination and detection assemblies and a plurality of pressure assemblies.

The method may include wirelessly communicating between the feedback unit and the illumination and detection assembly and pressure assembly.

The detected signal may be a photoplethysmography (PPG) signal.

In some example embodiments, a computer program product is provided for detecting a physiological signal using an optical measurement device, the computer program product embodied on a computer readable medium and when executed by a computer with a processor and a memory, performs the method comprising: illuminating a portion of living tissue of a user and detecting transmitted or reflected light as a signal using an illumination and detection assembly; detecting an amount of pressure applied by the portion of living tissue of the user to the illumination and detection assembly; correlating the quality of the detected signal with the amount of applied pressure; and providing feedback related to the correlation to the user.

In an example, there is provided an optical measurement device comprising an illumination and detection assembly configured to output light to a surface portion of a user for measurement and to detect the output light transmitted or reflected from said surface portion of the user as a signal; a pressure assembly configured to detect an amount of pressure applied to the illumination and detection assembly by the user; wherein the amount of pressure detected is capable of being used as feedback information to the user.

The illumination and detection assembly and pressure assembly may be integrated into a single portable device and a feedback unit may be further provided separately.

The pressure assembly may not substantially deform or displace when in use.

The device may be a reflectance-based device.

The illumination and detection assembly may comprise a red light emitting diode (LED), an infra-red LED or both.

Saturation of peripheral oxygen (SPO2) information of the user may be derivable from the detected output light information from both the red LED and the infra-red LED.

The optical measurement device may further comprise a coupling member configured for coupling in a cableless configuration to a personal mobile processing device.

The feedback unit may be configured to correlate the quality of the detected signal with the amount of applied pressure and provide feedback related to the correlation to the user.

The illumination and detection assembly, pressure assembly and feedback unit may be integrated into a portable device.

The personal mobile processing device may comprise the feedback unit configured to correlate the quality of the detected signal with the amount of applied pressure and provide feedback related to the correlation to the user.

The feedback may be an indication of whether the user should adjust the amount of pressure being applied to the illumination and detection assembly.

The feedback may display a range of optimal applied pressures along with the actual applied pressure being applied by the user.

The range of optimal applied pressures may correspond to a state of zero transmural pressure.

The feedback may be a request to the user to increase, decrease or maintain the applied pressure.

The feedback may be a real-time visual output of the detected signal and detected applied pressure.

The feedback unit may be a portable computer including a processor, memory and a display.

The portable device may be configured with a plurality of illumination and detection assemblies and a plurality of pressure assemblies.

The illumination and detection assembly and the pressure assembly may communicate with the feedback unit over a wireless network.

The detected signal may be a photoplethysmography (PPG) signal.

The personal mobile processing device may be one selected from a group consisting of a mobile phone, a smartphone, a personal digital assistant (PDA), a mobile music player, a tablet computer, a netbook and a laptop.

In an example, there is provided a method for detecting a physiological signal using an optical measurement device, comprising illuminating a surface portion of a user for measurement and detecting transmitted or reflected output light from said surface portion of the user as a signal using an illumination and detection assembly; detecting an amount of pressure applied by the user to the illumination and detection assembly using a pressure detection assembly; wherein the amount of pressure detected is capable of being used as feedback information to the user.

The illumination and detection assembly and pressure detection assembly may be integrated into a single portable device and a feedback unit may be further provided separately.

The pressure assembly may not substantially deform or displace when in use.

The measurement device may be a reflectance-based device.

The illumination and detection assembly may comprise a red light emitting diode (LED), an infra-red LED or both.

The method may further comprise deriving saturation of peripheral oxygen (SPO2) information of the user from the detected output light information from both the red LED and the infra-red LED.

The method may further comprise coupling a coupling member in a cableless configuration to a personal mobile processing device.

The method may further comprise correlating the quality of the detected signal with the amount of applied pressure; and providing feedback related to the correlation to the user using the feedback unit.

The method may further comprise providing an indication to the user of whether the amount of pressure being applied to the illumination and detection assembly should be adjusted.

The method may further comprise displaying a range of optimal applied pressures along with the actual applied pressure being applied by the user.

The method may further comprise displaying a range of optimal applied pressures along which corresponds to a state of zero transmural pressure.

The method may further comprise requesting the user to increase, decrease or maintain the applied pressure.

The method may further comprise displaying a real-time visual output of the detected signal and the detected applied pressure.

The feedback unit may be a portable computer including a processor, memory and a display.

The illumination and detection assembly, pressure assembly and feedback unit may be integrated into a portable device.

The portable device may be configured with a plurality of illumination and detection assemblies and a plurality of pressure assemblies.

The method may further comprise wirelessly communicating between the feedback unit and the illumination and detection assembly and pressure assembly.

The method may further comprise correlating the quality of the detected signal with the amount of applied pressure; and providing feedback related to the correlation to the user using a feedback unit; wherein the personal mobile processing device comprises the feedback unit.

The detected signal may be a photoplethysmography (PPG) signal.

In an example, there is provided a computer readable medium having stored thereon instructions for instructing a processor of an optical measurement device to execute a method for detecting a physiological signal using the optical measurement device, the method comprising illuminating a surface portion of a user for measurement and detecting transmitted or reflected output light from said surface portion of the user as a signal using an illumination and detection assembly; detecting an amount of pressure applied by the user to the illumination and detection assembly using a pressure detection assembly; wherein the amount of pressure detected is capable of being used as feedback information to the user.

The illumination and detection assembly and pressure detection assembly may be integrated into a single portable device and a feedback unit may be further provided separately.

The pressure assembly may not substantially deform or displace when in use.

The illumination and detection assembly may comprise a red light emitting diode (LED), an infra-red LED or both.

The method of the medium may further comprise deriving saturation of peripheral oxygen (SPO2) information of the user from the detected output light information from both the red LED and the infra-red LED.

The method of the medium may further comprise correlating the quality of the detected signal with the amount of applied pressure; and providing feedback related to the correlation to the user using the feedback unit.

In an example, there is provided an accessory for a personal mobile processing device, the accessory comprising a pressure assembly configured to detect an amount of pressure applied to an illumination and detection assembly of the personal mobile processing device by a surface portion of a user for measurement.

The pressure assembly may not substantially deform or displace when in use.

The accessory may be removably attached to a casing for the personal mobile processing device.

The accessory may be integrally attached to a casing for the personal mobile processing device.

The personal mobile processing device may be one selected from a group consisting of a mobile phone, a smartphone, a personal digital assistant (PDA), a mobile music player, a tablet computer, a netbook and a laptop.

It is to be understood that both the foregoing and the following descriptions are exemplary and explanatory only and are not intended to limit the claimed invention or application thereof in any manner whatsoever.

### BRIEF DESCRIPTION OF THE DRAWINGS

The accompanying drawings, which are incorporated in and constitute a part of this specification, exemplify the embodiments of the present invention and, together with the description, serve to explain and illustrate principles of the invention. Specifically:
FIG. 1 is an illustration of a photoplethysmograph (PPG) and the components thereof, as is conventional in the art;
FIG. 2 is an illustration of an AC pulse waveform of the PPG, as is conventional in the art;
FIG. 3 is an illustration of a cross-section of a blood vessel when a low external pressure is applied;
FIG. 4 is an illustration of the cross-section of the blood vessel when a high external pressure is applied;
FIG. 5 is a graphical illustration of an amplitude of a PPG signal received during increasing amounts of external pressure in a state of zero transmural pressure;
FIG. 6A is an illustration of an optical measurement device, according to an exemplary embodiment;
FIG. 6B is an exploded view illustration of the optical measurement device, including an illumination and detection assembly and a pressure detection assembly, according to an exemplary embodiment;
FIGs. 7A and 7B are expanded view illustrations of the illumination and detection assembly and a pressure detection assembly of the optical measurement device and a method of use with a human finger, and according to one exemplary embodiment;
FIG. 8 illustrates a graphical comparison of a graph of measured voltage of a PPG signal over time as it corresponds to a graph of an applied amount of pressure over time;
FIGs. 9A and 9B illustrate a feedback unit, such as a portable device with a display, in connection with an optical measurement device and a user's interaction therewith, according to an exemplary embodiment;
FIG. 10 illustrates a graphical user interface (GUI) on the display, including a graphical representation of a PPG signal and a graphical representation of applied pressure, according to an exemplary embodiment;
FIG. 11 illustrates a portable device integrated with an optical measurement device, according to an exemplary embodiment;
FIG. 12 illustrates an optical measurement device integrated with a touchscreen display of a portable device, according to an exemplary embodiment;
FIGs. 13A and 13B illustrate a portable device connected with an optical measurement device configured in a landscape orientation and a user's interaction therewith, according to an exemplary embodiment;
FIGs. 14A and 14B illustrate a portable device integrated with a plurality of optical measurement devices in a landscape orientation and a user's interaction therewith, according to an exemplary embodiment;
FIGs. 15A and 15B illustrate a portable device integrated with a plurality of optical measurement devices located on a side portion of the portable device, according to an exemplary embodiment;
FIGs. 16A and 16B are expanded view illustrations of an alternate embodiment of a method of using the optical measurement device with the human finger to detect the blood pressure of the human, according to one exemplary embodiment;
FIG 17 is a block diagram of the optical measurement device, according to an exemplary embodiment;
FIGs 18A, 18B and 18C are graphical illustrations of signals used in the process of obtaining a direct current (DC) component of the PPG signal, according to an exemplary embodiment;

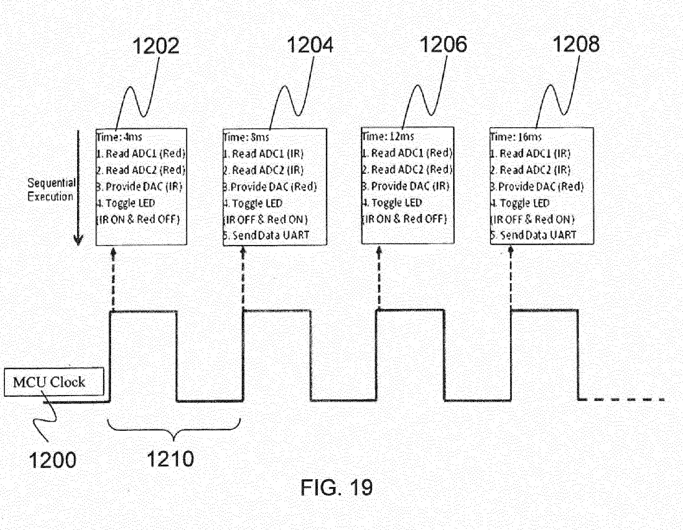

FIG. 19 is an illustration of a sequence of data collection performed during the process of obtaining the PPG signal, according to an exemplary embodiment;
FIG. 20 is a flow chart illustrating a method of measuring the PPG signal on the optical measurement device using feedback from the pressure detection assembly, according to an exemplary embodiment;
FIGs. 21A, 21B and 21C are graphical representations of the correlation between a PPG waveform and an applied pressure, as would be used in the method of measuring an optimal PPG signal, according to an exemplary embodiment;
FIG. 22 is a block diagram of a computer system upon which the device and methods may be implemented, according to an exemplary embodiment;
FIG. 23 is a schematic flowchart illustrating a method for detecting a physiological signal using an optical measurement device in an example embodiment; and
FIG. 24 shows a simplified exemplary representative circuit diagram for implementing a piezo-based sensing device in an example embodiment.
FIG. 25A is a cross-sectional view of a cantilever type optical measurement device in accordance with another embodiment disclosed herein.
FIG. 25B is an exploded view of the optical measurement device of FIG. 25A.
FIG. 25C is a simplified view of Fig. 25A showing only the main components that may be useful for transmittance and sensing of an exerted force.
FIG. 25D and FIG. 25E are schematics of an exemplary cantilever type force application.
FIG. 25F is a perspective view of the underside of the transparent plastic piece and the top portion of the plastic housing of FIG. 25B.
FIG. 25G is an exploded view of another exemplary embodiment of the optical measurement device disclosed herein.
FIG. 25H is a cross-sectional view of an assembled optical measurement device of FIG. 25A at various levels of stripping to emphasize the structure of the transparent plastic piece.
FIG. 25I is a cross-sectional view of an assembled optical measurement device of FIG. 25G, at various levels of stripping to emphasize the structure of the opaque or substantially opaque plastic piece.

### DETAILED DESCRIPTION

An optical measurement device for obtaining non-invasive physiological measurements and method of using the same is described more fully herein, the device comprising a pressure detection assembly configured to detect an amount of pressure applied by a body part of a user to the device during the optical measurement. When the user applies an appropriate amount of pressure to the optical measurement device, the resulting signal-to-noise ratio of the detected optical measurement signal can be increased, and a more accurate measurement signal can be obtained from the user. An optimal pressure can be determined in real-time by analyzing the detected optical measurement signal and correlating a high signal-to-noise ratio portion of the signal with a corresponding applied pressure. The user can be provided real-time feedback indicating whether the amount of pressure being applied by the user should be increased, decreased or maintained at the same level. The optical measurement device can therefore facilitate in providing an optimum pressure determination customized for each individual user, thereby obtaining a resulting optimum measurement signal for each user.

The description herein may be, in certain portions, explicitly or implicitly described as algorithms and/or functional operations that operate on data within a computer memory or an electronic circuit. These algorithmic descriptions and/or functional operations are usually used by those skilled in the information/data processing arts for efficient description. An algorithm is generally relating to a self-consistent sequence of steps leading to a desired result. The algorithmic steps can include physical manipulations of physical quantities, such as electrical, magnetic or optical signals capable of being stored, transmitted, transferred, combined, compared, and otherwise manipulated.

Further, unless specifically stated otherwise, and would ordinarily be apparent from the following, a person skilled in the art will appreciate that throughout the present specification, discussions utilizing terms such as "scanning", "calculating", "determining", "replacing", "generating", "initializing", "outputting", and the like, refer to action and processes of a instructing processor/computer system, or similar electronic circuit/device/component, that manipulates/processes and transforms data represented as physical quantities within the described system into other data similarly represented as physical quantities within the system or other information storage, transmission or display devices etc.

The description also discloses relevant device/apparatus for performing the steps of the described methods. Such apparatus may be specifically constructed for the purposes of the methods, or may comprise a general purpose computer/processor or other device selectively activated or reconfigured by a computer program stored in a storage member. The algorithms and displays described herein are not inherently related to any particular computer or other apparatus. It is understood that general purpose devices/machines may be used in accordance with the teachings herein. Alternatively, the construction of a specialized device/apparatus to perform the method steps may be desired.

In addition, it is submitted that the description also implicitly covers a computer program, in that it would be clear that the steps of the methods described herein may be put into effect by computer code. It will be appreciated that a large variety of programming languages and coding can be used to implement the teachings of the description herein. Moreover, the computer program if applicable is not limited to any particular control flow and can use different control flows without departing from the scope of the invention.

Furthermore, one or more of the steps of the computer program if applicable may be performed in parallel and/or sequentially. Such a computer program if applicable may be stored on any computer readable medium. The computer readable medium may include storage devices such as magnetic or optical disks, memory chips, or other storage devices suitable for interfacing with a suitable reader/general purpose computer. The computer readable medium may even include a wired medium such as exemplified in the Internet system, or wireless medium such as exemplified in bluetooth technology. The computer program when loaded and executed on a suitable reader effectively results in an apparatus that can implement the steps of the described methods.

The example embodiments may also be implemented as hardware modules. A module is a functional hardware unit designed for use with other components or modules. For example, a module may be implemented using digital or discrete electronic components, or it can form a portion of an entire electronic circuit such as an Application Specific Integrated Circuit (ASIC). A person skilled in the art will understand that the example embodiments can also be implemented as a combination of hardware and software modules.

In the following detailed description, reference will be made to the accompanying drawings. The aforementioned accompanying drawings show by way of illustration and not by way of limitation, specific embodiments and implementations consistent with principles of the present invention. The following detailed description is, therefore, not to be construed in a limited sense. Additionally, the various embodiments of the invention as described may be implemented in the form of software running on a general purpose computer, in the form of a specialized hardware, or combination of software and hardware.

In the following description, it will be appreciated that the optical measurement device described can be, but is not limited to, a reflectance-based measurement device. In some example embodiments, saturation of peripheral oxygen (SPO2) information of a user is derivable from detected output light information from both a red LED and an infra-red LED. In some example embodiments, the measurement device can function as an accessory to a personal mobile processing device.

Further, in the description herein, the term "light" as used herein is meant to be interpreted in a broad sense and is not limited to visible light only. The term "light" as used herein can include, but is not limited to, X-ray light rays, visible light rays, ultraviolet light rays and infra-red light rays.

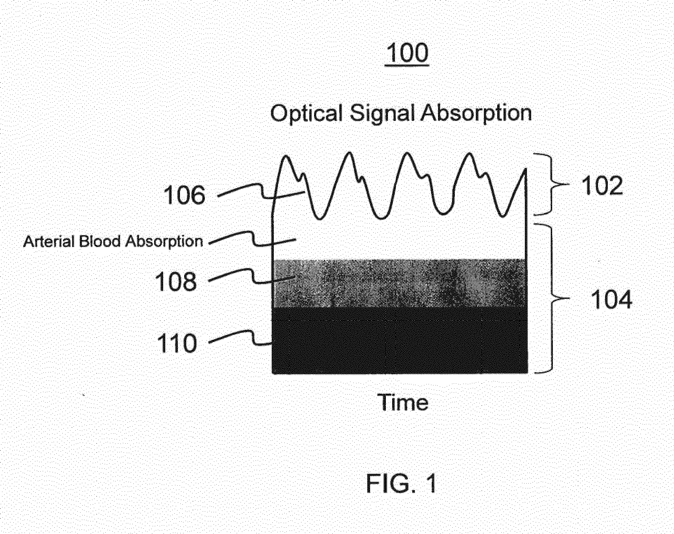

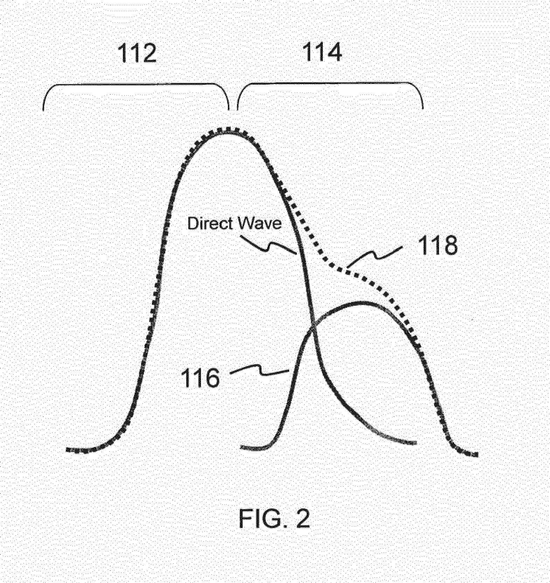

FIG.1 and FIG. 2 are provided to briefly describe components of a PPG signal. FIG. 1 illustrates a graphical representation of a photoplethysmograph (PPG) signal 100, which can generally be divided into two components: an AC component **102** due to the absorption of light in pulsatile arterial blood volume **106;** and a DC component **104** caused by the absorption produced by non-pulsatile arterial blood - i.e. venous blood and capillary blood **108,** and tissue absorption **110.**

In FIG. 1, this AC component **102** is superimposed onto a large quasi-DC component **104** that relates to the tissues and to the average blood volume. This DC component **104** varies slowly due to respiration, vasomotor activity and vasoconstrictor waves. With suitable electronic filtering and amplification, both the AC component **102** and DC component **104** can be extracted for subsequent pulse wave analysis.

Two significant characteristics of the PPG AC pulse waveform **102** have been described and are illustrated in FIG. 2, where the appearance of the pulse waveform was defined as two phases: a first anacrotic phase **112** being the rising edge of the pulse, and a second catacrotic phase **114** being the falling edge of the pulse. The first phase **112** is primarily concerned with systole, while the second phase **114** represents diastole and wave reflections **116** from the periphery. A dicrotic notch **118** is usually seen in the second catacrotic phase **114** of subjects with healthy compliant arteries.

As discussed, the acquisition of a physiological signal representing a change in the volume of an organ in the body through the use of optical measurement is known as a photoplethysmograph (PPG). Obtaining optical PPG signals typically requires application of external pressure on the body surface which is being measured. The pressure correlates to obtaining a good quality PPG signal with a high signal to noise ratio.

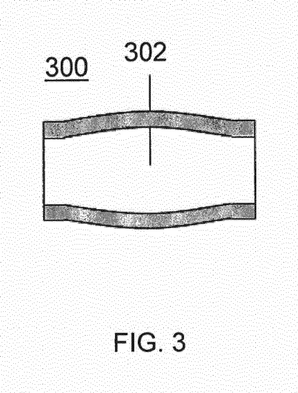

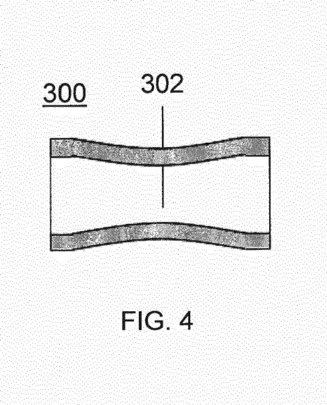

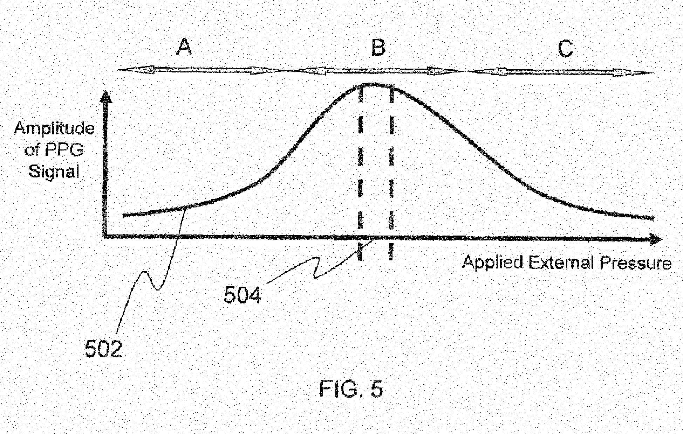

However, the externally-applied pressure cannot be too large or too small, or the quality of the detected PPG signal will be low. For example, as illustrated in a cross section of a blood vessel **300** in FIG. 3, in the event of an insufficient exertion of external force as compared to internal arterial pressure at a measurement site **302,** the internal pressure is too low to obtain a proper measurement, and low PPG signals are obtained. On the contrary, as illustrated in FIG. 4, the application of too much external force causes the blood vessel **300** to be occluded at the measurement site **302** where the pressure is applied, resulting in resistance of regular blood flow and generating skewed PPG signal data. If the external pressure is too small or too high, the reaction pressure at the wall of the blood vessel **300** is low, and thus a small PPG signal will be observed. FIG.5 is a graphical illustration of the amplitude **502** of a measured PPG signal in comparison with an amount of applied external pressure. With a low applied pressure in range A, the amplitude **502** is correspondingly low. As the applied pressure is increased, in range B, the amplitude also increases. However, when the applied pressure increases beyond a certain point, the amplitude decreases again, as shown in range C.

To obtain a strong PPG signal, the external pressure should be sufficient to minimize transmural pressure such that the external pressure is equal to the internal pressure. Further illustrated in FIG. 5 is a range **504** within range B where the amplitude of the PPG signal is at its peak. Within this range **504,** an externally-applied pressure is instantaneously balanced with the internal arterial pressure, thus resulting in a state of zero transmural pressure. At zero transmural pressure, the arterial walls are unloaded and the arteries will not change in size. Consequently, the blood volume within the arteries at the measured region will not change and can be accurately measured to provide a good quality PPG signal.

In an exemplary embodiment, the pressure assembly assists in achieving and maintainingan optimal pressure for obtaining an optimum PPG signal over an extended period of time. Preferably, by providing real-time, instantaneous feedback to a user being measured, the user can be able to instantly adjust the amount of pressure being applied to the device in order to obtain an optimum PPG signal. However, the optimum pressure may not only be a result of a state of zero transmural pressure, but may also result from the effects of absorption and scattering paths of light as light travels in and out of a portion of tissue of a user being measured. For example, where the pressure is too low, a light source may not be able to penetrate the tissue surrounding the blood vessel which is being measured. Therefore, light may not travel in and out of the finger effectively enough for a good PPG signal to be detected. Where the pressure is too high, light may be absorbed or scattered such that the amount of light detected is insufficient to obtain a good PPG signal.

In one exemplary embodiment, the device may assist in providing feedback to the user indicating whether the user is applying insufficient pressure, too much pressure or the correct amount of pressure. The feedback to the user may be visual or auditory in the form of a visual display or audible sounds, and may particularly be a display of the real-time PPG signal being captured by the device. The feedback may also be a more simplified display indicating whether the user should take action to increase or reduce the amount of pressure being applied to the device. In another embodiment, the feedback may be in the form of tactile feedback, wherein the device produces e.g. a small vibration when the applied pressure is at an optimum range.

Exemplary embodiments described herein seek to provide a device and method capable of augmenting signal to noise ratio in an optical signal of an illuminated region at a measuring site of a body part of a user. Exemplary embodiments also provide for detecting the optical response formed by both light reflected from the measuring site and the light transmitted through the measuring site. Exemplary embodiments described herein utilize redirecting reflections of light on its way towards the measuring site (i.e. blood vessels) back to the region of interest.

In an additional exemplary embodiment, the device may perform a series of calibration steps for each individual user in order to determine an optimum range of pressure for each individual. The subsequent steps of capturing the PPG signal will then use the predetermined optimum range as the benchmark for obtaining an optimum PPG signal.

### I. Device Overview

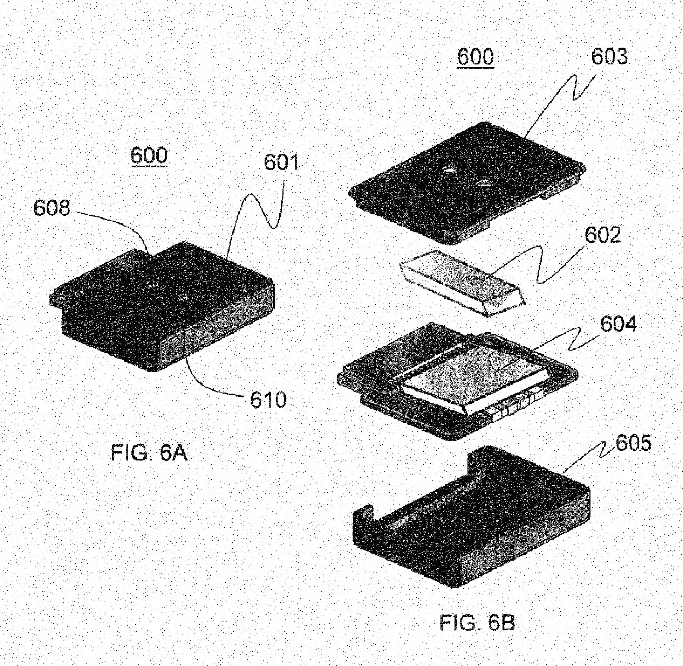

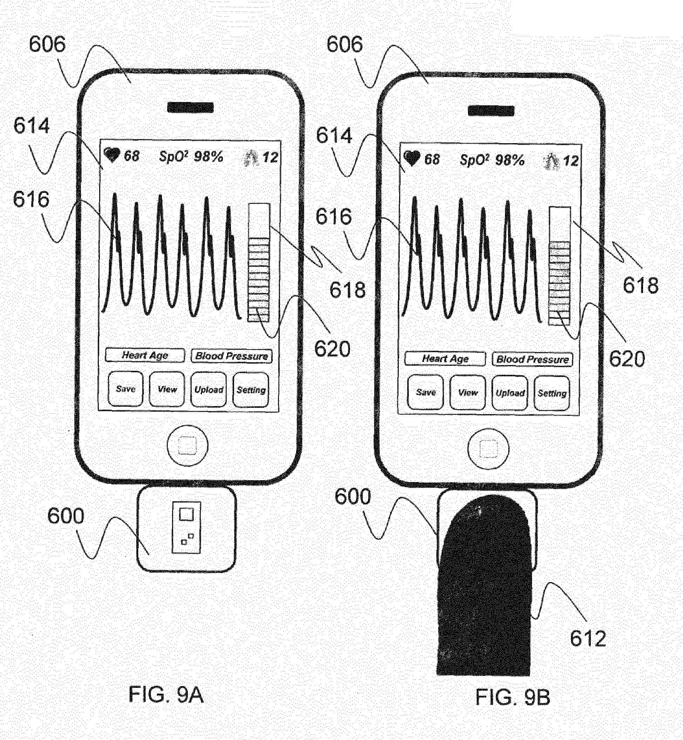

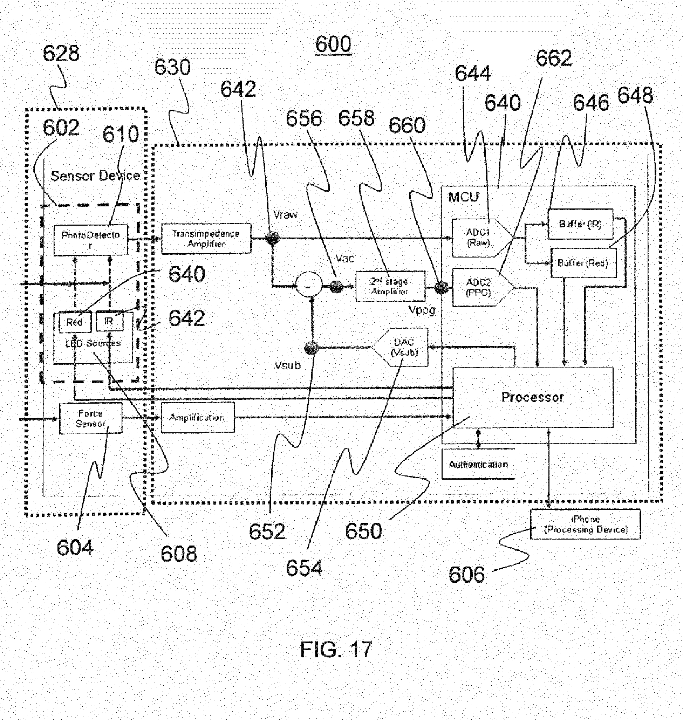

FIG. 6A illustrates one exemplary embodiment of an optical measurement device **600,** and FIG. 6B illustrates an exploded view of the optical measurement device **600,** showing the arrangement of an illumination and detection assembly **602** and a pressure detection assembly **604.** As illustrated in FIG. 6A, the illumination and detection assembly **602** and pressure detection assembly **604** may be integrated as a single, compact optical measurement device **600** surrounded by a housing **601** for portable use. In the exploded view in FIG. 6B, the housing **601** is shown divided into a top casing **603** and a base casing **605,** with the illumination and detection assembly **602** and pressure detection assembly **604** enclosed therein. The integration of the pressure detection assembly **604** with the illumination and detection assembly **602** provides a simple, comfortable interaction for the user. In one embodiment, the pressure detection assembly **602** comprises non-movable parts such that the pressure detection assembly **602** does not employ spring/elastic force and does not substantially deform or displace when in use. That is, when an external pressure or force is applied by the user to the pressure detection assembly, the pressure detection assembly does not substantially deform or displace. The use of a pressure detection assembly **604** in cooperation with e.g. an external feedback unit can provide real-time feedback to the user that improves the quality, or amplitude, of the received PPG signals. The optical measurement device **600** is connected with a feedback unit **606** (see FIG. 9A), which receives the PPG signals and pressure measurements from the optical measurement device **600** and provides feedback to the user regarding the amount of pressure being applied. Accordingly, in this embodiment, the feedback unit **606** can be provided separately from the optical measurement device **600.** The illumination and detection assembly **602** may be referred to as a PPG sensor, and includes a light source **608** and a plurality of light detectors **610** (see FIG. 17), where the light source **608** propagates light through a portion of living tissue at a measurement site of a user. The light detectors **610** then detect light which is transmitted through the portion of living tissue of the user or which is reflected from the portion of living tissue of the user. In one exemplary embodiment, the pressure detection assembly **604** is a pressure sensor that detects the amount of pressure that has been applied by a body part of the user, such as a finger. The pressure sensor may be a thin film flexible printed circuit, such as a piezo-based or piezoresistive sensing device whereby a resistance change sensed by the circuitry is inversely proportional to a change in force applied on the sensing device. In some example embodiments, the circuit is a micro-electro-mechanical (MEMS) strip. Nevertheless, any other force measuring device that is capable of sensing an applied contact force may be used. Preferably, the pressure sensor comprises non-movable parts, does not employ spring/elastic force and does not substantially deform or displace when in use.

In certain embodiments, the pressure detection assembly of the optical measurement device may comprise a cantilever means coupled to a force transmitting member for transmitting an exerted force by a cantilever moment to the force transmitting member. The cantilever means may be a beam-like structure supported by at least one fulcrum. In one embodiment, when in use, the fulcrum is disposed at one end of the beam-like structure. The cantilever means may also comprise a mating means that is capable of mating with a complementary matching means of a housing of the optical measurement device. The mating means of the cantilever means may be any physical feature that is capable of mating with the matching means of the housing so that the cantilever means can fit snugly within the housing. Likewise, the matching means of the housing may be any physical feature that is capable of mating with the mating means of the cantilever means to provide secure engagement therewith. In one embodiment, the mating means comprises one or more features selected from the group consisting of a protrusion, projection, abutment, extension and the like, while the matching means comprises one or more features selected from the group consisting of a hole, slot depression, recess, opening, aperture and the like. In another embodiment, the matching means comprises one or more features selected from the group consisting of a protrusion, projection, abutment, extension and the like, while the mating means comprises one or more features selected from the group consisting of a hole, slot, depression, recess, opening, aperture and the like. Preferably, both the mating means and matching means are stepped structures that are complementary to each other. In other embodiments, the cantilever means may be part of the force transmitting member and vice versa such that both the cantilever means and the force transmitting member form a single unitary structure.

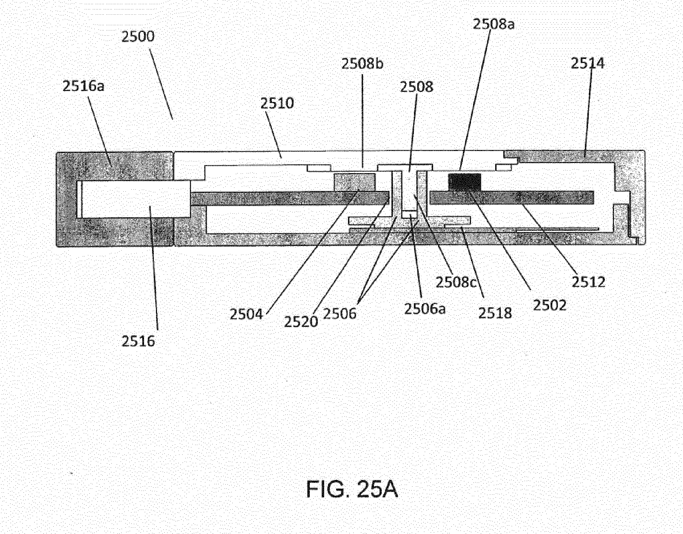

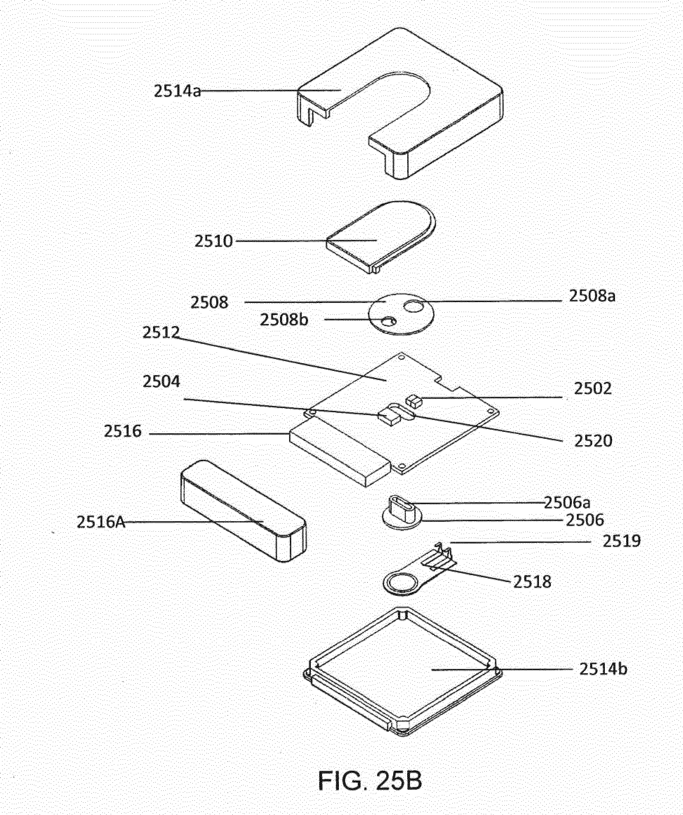

FIG. 25A is a cross-sectional view of a cantilever type optical measurement device in accordance with the invention.
The optical measurement device **2500** comprises a wave emitter in the form of a light emitting diode **2502** and a wave detector in the form of a photodetector **2504** disposed on the same horizontal plane (relative to the sensing device base) and on the same substrate, which is in the form of a printed circuit board **2512.** The wave emitter and the wave detector may be part of the illumination detection assembly. The substrate or printed circuit board **2512** further comprises an opening **2520** disposed between the LED **2502** and photodetector **2504.** A force transmitting member comprising a plastic assembly piece **2506** and an overhead portion **2508,** is arranged such that the plastic assembly piece **2506** extends through the opening **2520,** between the light emitting diode **2502** and the photodetector **2504.** The force transmitting member may be part of a pressure assembly. The overhead portion **2508** is provided to increase the overall surface area available to substantially prevent light emitted by the LED **2502** from travelling directly to the photodetector **2504.** In this exemplary embodiment, the overhead portion **2508** is inserted into the plastic assembly piece such that both of them fit snuggly with each other. The overhead portion **2508** can also be provided in other forms, shapes and/or orientation so long as it serves to substantially prevent light emitted by the LED **2502** from travelling directly to the photodetector **2504.** The overhead portion **2508** can be a detachable portion that is attached to the plastic assembly piece **2506.** Alternatively, the overhead portion **2508** can be a continuous part of the plastic assembly piece **2506** formed as a single structure. The plastic assembly piece **2506** is opaque and substantially does not allow light from the light emitting diode **2502** to travel directly to the photodetector **2509.** The overhead portion **2508** is also opaque. However, the overhead portion **2508** comprises access hole **2508a** for allowing light emitted from the light emitting diode **2502** to pass through and access hole **2508b** for allowing reflected light to pass through to reach the photodetector **2504.** As can be seen in Fig 25A, the force transmitting member **2506** as a whole appears to have a forked shaped cross sectional area to allow the overhead portion **2508** to rest thereon. The forked shaped cross sectional area provides stability to the overhead portion **2508** and also facilitates transmission of an exerted force via a cantilever type moment. From the side view, the forked shape cross sectional area appears to comprise a base having a plurality of walls (for e.g. two walls) extending from the base such that the walls are substantially perpendicular to the base. From the side view, the walls are spaced at a distance from each other. From a three-dimensional perspective, the force transmitting member **2506** can in fact be a circumferential wall extending from a base having the forked shaped cross sectional area described above. The circumferential wall may be continuously joined such that it forms a enclosure around a space on the base (as shown for example in component **2506** of FIG 25B). The circumferential wall can be annular shaped or in any other shape as long as it forms an enclosure around a space on the base. In other embodiments, it is also possible that the circumferential wall comprises one or more gaps to disrupt its continuity but may nevertheless still be considered to generally surround a space on the base. Due to the wall/s extending from the base, the space on the base that is surrounded by the wall/s may be deemed as a recess or cavity. In some other cases, the walls may be deemed as a built up area around the space on the base. The overhead portion **2508** can have a protrusion **2508c** that is complementary to this recess or cavity such that the protrusion of the overhead portion **2508** can be inserted into the recess or cavity **2506a** within the plastic assembly piece **2506** to provide a more snug or rigid fit. It will be appreciated that in some exemplary embodiments this complementary mating structure may be reversed such that the recess or cavity now exists on the overhead portion and the protrusion exists on the force transmitting member.

The positioning and the structure of the force transmitting member **2506** substantially prevents light emitted by the light emitting diode **2502** from traveling directly to the photodetector **2509.** The force transmitting member is rested on a sensing substrate in the form of force sensor **2518.** The force sensor **2518** can be provided as a flexible printed circuit that senses contact force and provides the associated electrical signal to the printed circuit board **2512** via the electrical connectors at one end of the force sensor **2518** (not shown in FIG 25A.). Due to its flexibility, the force sensor **2518** is able to deform slightly when the force transmitting member transmits a force to the metal connector **2518.** The optical measurement device **2500** also comprises a plastic housing **2514** for housing the individual components described above. At the top of the housing **2514,** there is provided an orientation-free measurement surface in the form of a transparent plastic piece **2510** for receiving a surface portion of the user to be detected. The orientation-free measurement surface in the form of the transparent plastic piece **2510** is not limited to a single orientation at which the surface portion of the user must be placed. For example, as compared to a clip or a cuff, the orientation-free measurement surface does not require the surface portion of the user to be engaged therewith in a particular fashion, so long as the user surface in contact with the orientation-free measurement surface is capable of reflecting the emitted waves towards the wave detector. As such, the orientation-free measurement surface is capable of advantageously detecting a 2-dimensional surface. On the other hand, the portion of the user to be detected must be 3-dimensional when a clip or a cuff is used, in order to ensure sufficient engagement with the clip or the cuff for detection. The transparent plastic piece 2510 may further provide an additional layer of protection, to prevent direct contact and damage to the LED and photodetector. It may also serve to prevent dust and small particles from entering the housing of the sensing device.

In this exemplary embodiment, the optical measurement device **2500** also comprises a coupling member in the form of a data communication port **2516** electrically coupled to the printed circuit board **2512.** The data communication port **2516** is capable of transmitting electrical signals to and from the sensing device **2500.** The data communication port **2516** is also capable of transmitting electrical power to power the printed circuit board **2512** and its electrically connected components such as the light emitting diode **2502** and the photodetector **2504** and force sensor **2518.**

In use, the sensing device **2500** can be connected to a personal mobile processing unit for example a mobile phone via the data communication port **2516.** The user then places a desired surface to be detected, for example a finger, onto the transparent plastic piece **2510.** Light emitted from the light emitting diode **2502** travels through the access hole **2508a** and towards the finger surface in contact with the transparent plastic piece **2510.** The emitted light that is reflected from the finger surface passes through access hole **2508b** and towards the photodetector **2504.** The photodetector **2504** then transmits an electrical signal representative of the detected reflected light to the mobile phone via the data communication port **2516.** A cover **2516A** may be provided for the data communication port **2516.** At the same time, the exerted force is transmitted via the plastic assembly piece 2506 towards the force detector 2518. The force detector **2518** then provides an electrical signal representative of the force with the circuit board **2512.** The electrical signal is then transmitted to the mobile phone via the data communication port **2516.** The mobile phone may comprise a processing unit to process the signals received from the optical measurement device **2500.** The mobile phone may also comprise a feedback unit to indicate to the user whether the force exerted by the finger is too high or low. The user may then adjust the force or pressure accordingly and once the optimum pressure is detected, the mobile phone will display the physiological characteristics that are derived from the properties of the reflected light detected.

FIG. 25B is an exploded view of the sensing device of FIG. 25A. The individual components are dismantled and can be clearly seen. The plastic housing **2514** can be seen to be separated into a top portion **2514a** and a bottom portion **2514b.** The force sensor **2518** senses contact force and provides the associated electrical signal to the printed circuit board **2512** via the electrical connectors **2519** at one end of the force sensor **2518.**

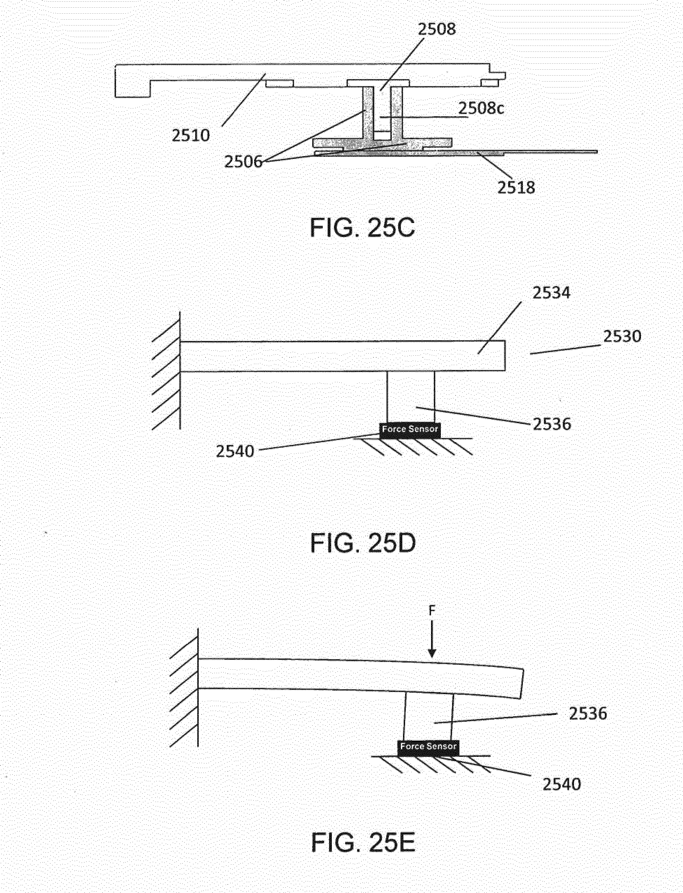

FIG. 25C is a simplified view of Fig. 25A showing only the main components that may be useful for the transmission and the sensing of an exerted force through a cantilever type moment. The cantilever structure can restrict movement of the column in all direction except one. This can give the column better stability and easy for user to maintain a constant applied force.

FIG. 25D and FIG. 25E show schematics of an exemplary cantilever type force application **2530.** Component **2534** can be taken to be a simplified representation of the transparent plastic piece **2510** together with the overhead portion **2508** of FIG 25B, Component **2536** can be taken to be a simplified representation of the force transmitting member **2506** of FIG 25B, and Component **2540** can be taken to be a simplified representation of the force detector **2518** of FIG 25B. When force F is applied on Component **2534** towards Component **2536,** a bending moment occurs resulting in deformation of Component **2534** and Component **2536** towards one direction. This bending moment is transmitted to Component **2540** for detection.

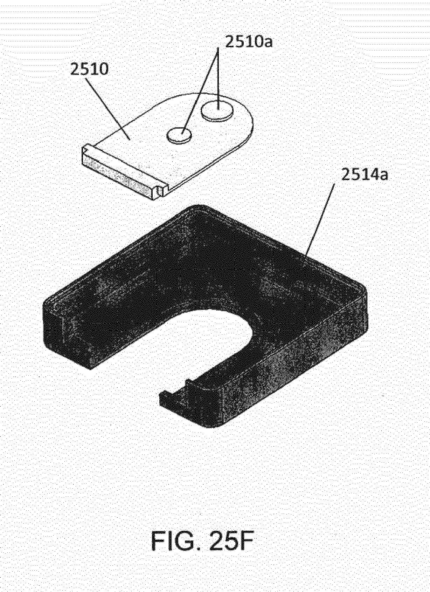

FIG. 25F is a perspective view of the underside of the transparent plastic piece **2510** and the top portion of the plastic housing **2514a** of FIG. 25B. As shown in FIG. 25F, the transparent plastic piece **2510** comprises protrusions **2510a** that are able to fit into the access holes **2508a** and **2508b** of the overhead portion **2508** of FIG. 25B in order to secure and prevent undesirable lateral movement of the transparent plastic piece **2510.**

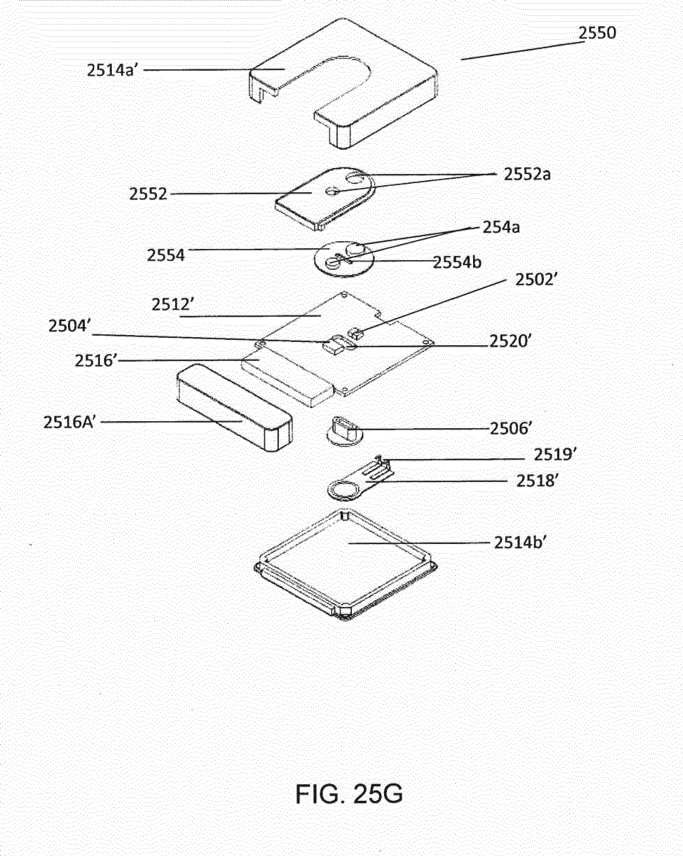

FIG. 25G is an exploded view of another exemplary embodiment of an optical measurement device **2550** disclosed herein. In this exemplary embodiment, most of the components are similar to those described in FIG 25B. These similar elements are labelled with the same reference numerals used in FIG. 25B but with the inclusion of the prime symbol '. In this exemplary embodiment, the main differences from the embodiment of FIG. 25B are the components **2552** and **2554.** Component **2552** is a plastic piece that is opaque or substantially opaque (allows minimal light to pass through) but has access holes **2552a** to allow light to pass through. Component **2554** is an overhead portion similar to the overhead portion **2508** of FIG. 2B. However, component **2554** is substantially transparent which can allow light to pass through. Further, protrusions **2554a** (which are also transparent), are present instead of the access holes **2508a** and **2508b** shown in FIG. 25B. The protrusion **2554a** are able to fit into the access holes **2552a** of the opaque or substantially opaque plastic piece **2552** in order to secure and prevent undesirable lateral movement of the plastic piece **2552.** It will be appreciated that this configuration is reversed from that shown in FIG. 25F.

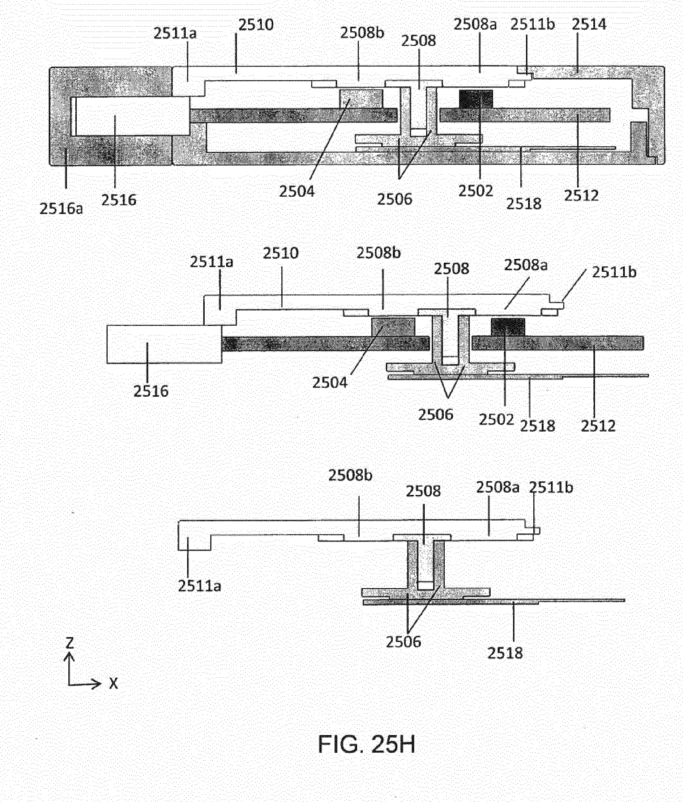

FIG. 25H is a cross-sectional view of an assembled sensing device of FIG. 25A at various levels of stripping to emphasize the structure of the transparent plastic piece. As shown in FIG. 25H, the transparent piece **2510** has a fulcrum end **2511a** that fits snugly into the casing **2514,** such that lateral movements in the plane defined by the X and Y coordinate axes (the Y axis being the axis going into the page and the X axis being the axis going from the left to the right of the page from the viewer's perspective) are restricted. The opposite end **2511b** is free to move downwards along the Z coordinate axis (the Z axis being the axis going from bottom to top of the page from the viewer's perspective). However, the transparent piece **2510** has a stepped outer edge such that the bottom surface is wider than its top surface. The casing **2514** is correspondingly dimensioned to only fit the smaller top surface, but not the bottom surface. Therefore, because the larger bottom surface is restricted from moving out of the casing surface, upward movement of the cantilever transparent piece cannot exceed the default position. When attempts are made to move the cantilever transparent piece **2510** upwards, the stepped edge at the opposite end 2511b engages with a complementary stepped edge of casing **2514,** thus arresting upward movement from its predetermined position. This can prevent the transparent piece **2510** from falling out of the casing **2514** and can also allow the measurement of an accurate default pressure, where no external force is applied. In certain embodiments, a resilient means such as a spring can be coupled to the stepped edge at the opposite end **2511b** to return it to its original configuration when a user exerted downward force on it is removed. The resilient means can also urge the stepped edge at the opposite end **2511b** towards the casing **2514** for engagement thereto, when the user exerted force on opposite end is removed. When fully assembled, the fulcrum end **2511a** of the transparent plastic piece **2510** (acting as a cantilever) can sit on top of the communication port **2516,** as shown in FIG. 25H. In certain embodiments, a resilient means such as a spring can be present between the transparent plastic piece **2510** and the communication port **2516.** It can be seen in FIG. 25H that the transparent plastic piece **2510** is perched at the end of the communication port **2516** such that part of **2511a** is free and not in contact with the communication port **2516.** This can possibly allow some clockwise rotation (from the viewer's perspective of FIG. 25H) of the plastic piece **2510** when a force is applied at the opposite end. As the transparent plastic piece **2510** can also act as a cantilever, the transparent plastic piece **2510** can be substantially flexible but at the same time have sufficient flexural strength to withstand the force exerted by the user during a PPG measurement. The transparent plastic piece **2510** may also have sufficient flexural strength and/or elasticity to return it back to its original shape/position when the exerted force is removed. In some other exemplary embodiments, the transparent plastic piece **2510** may be coupled to a resilient means such as a spring which returns the transparent plastic piece **2510** back to its original configuration once the exerted force is removed. With complementary mating structures present among the different components, it will be appreciated that the exemplary sensing device can be easily assembled to form a rigid and stable assembly which arrests undesired movement. This can allow the sensing device to have a high level of measurement accuracy and consistency by reducing the number of unknown or variable parameters present (for e.g. caused by undesired movement of the components). Another advantage of the complementary mating structures would be that the individual components can be reversibly assembled or disassembled from each other, making repairs or replacement of the individual components convenient. It will also be appreciated that the exemplary sensing device with its individual complementary mating structures can be adapted into a snap-fit design for easier assembly and disassembly.

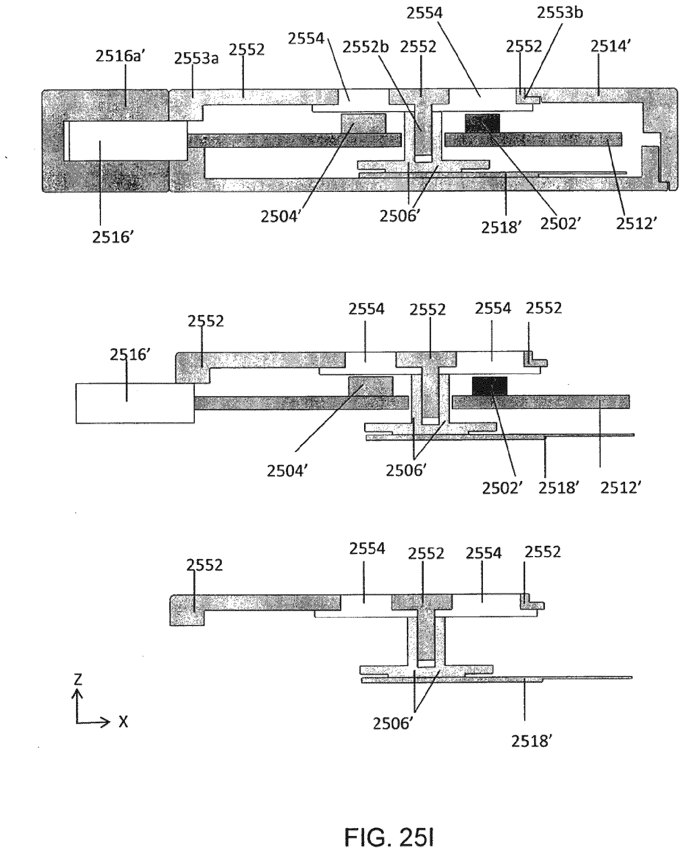

FIG. 25I is a cross-sectional view of an assembled sensing device of FIG. 25G , at various levels of stripping to emphasize the structure of the opaque or substantially opaque plastic piece. As shown in FIG. 25I, the plastic piece **2552** has a fulcrum end **2553a** that fits snugly into the casing **2514',** such that lateral movements in the plane defined by the X and Y coordinate axes (the Y axis being the axis going into the page and the X axis being the axis going from the left to the right of the page from the viewer's perspective) are restricted. The opposite end **2553b** is free to move downwards along the Z coordinate axis (the Z axis being the axis going from bottom to top of the page from the viewer's perspective). However, the plastic piece **2552** has a stepped outer edge such that the bottom surface is wider than its top surface. The casing **2514'** is correspondingly dimensioned to only fit the smaller top surface, but not the bottom surface. Therefore, because the larger bottom surface is restricted from moving out of the casing surface, upward movement of the cantilever piece cannot exceed the default position. When attempts are made to move the cantilever plastic piece **2552** upwards, the stepped edge at the opposite end **2553b** engages with a complementary stepped edge of casing **2514',** thus arresting upward movement from its predetermined position. This can prevent the plastic piece **2552** from falling out of the casing **2514'** and can also allow the measurement of an accurate default pressure, where no external force is applied by the user. In certain embodiments a resilient means such as a spring can be coupled to the stepped edge at the opposite end **2553b** to return it to its original configuration when a user exerted downward force on it is removed. The resilient means can also urge the opposite end **2553b** towards the casing **2514'** for engagement thereto, when the user exerted force on the opposite end 2553b is removed. The cantilever piece 2552 comprises a further center protrusion **2552b** which can extend through an opening **2554b** (See Fig. 25G) of the overhead transparent piece **2554** and be inserted into a complementary recess or cavity within the plastic assembly piece **2506'** to provide a more snug or rigid fit. When fully assembled, the fulcrum end **2553a** of the plastic piece **2554** (acting as a cantilever) can sit on top of the communication port **2516',** as shown in FIG. 25I. In certain embodiments a resilient means such as a spring can be present between the plastic piece **2552** and the communication port **2516'.** It can be seen in FIG. 25I that the transparent plastic piece **2552** is perched at the end of the communication port **2516'** such that part of **2553a** is free and not in contact with the communication port **2516'.** This can possibly allow some clockwise rotation (from the viewer's perspective of FIG. 25I) of the plastic piece **2552** when a force is applied at the opposite end. As the plastic piece **2552** can also act as a cantilever, the plastic piece **2552** can be substantially flexible but at the same time have sufficient flexural strength to withstand the force exerted by the user during a PPG measurement. The plastic piece **2552** may also have sufficient flexural strength and/or elasticity to return it back to its original shape/position when the exerted force is removed. In some other exemplary embodiments, the plastic piece **2552** may be coupled to a resilient means such as a spring which returns the plastic piece **2552** back to its original configuration once the exerted force is removed. With complementary mating structures present among the different components, it will be appreciated that the exemplary sensing device can be easily assembled to form a rigid and stable assembly which arrests undesired movement. This can allow the sensing device to have a high level of measurement accuracy and consistency by reducing the number of unknown or variable parameters present (for e.g. caused by undesired movement of the components). Another advantage of the complementary mating structures would be that the individual components can be reversibly assembled or disassembled from each other, making repairs or replacement of the individual components convenient. It will also be appreciated that the exemplary sensing device with its individual complementary mating structures can be adapted into a snap-fit design for easier assembly and disassembly.

While in the above description, the measurement surface (which can be a transparent plastic piece or an opaque plastic piece), the overhead portion and the force transmitting member appear to be described as separate components, they may in some embodiments be taken to be parts of a single overall functional component that achieves the cantilever moment when a force is exerted. Therefore, in some embodiments, the plastic piece, the overhead portion and the force transmitting member may collectively form a single unitary structure, which as a whole, may be considered to a cantilever structure.

In addition, while it has been described above that the wave emitter may comprise a LED, in some embodiments, a wave emitter may comprise a plurality of LEDs at least one being a red LED and one being an infra-red LED.

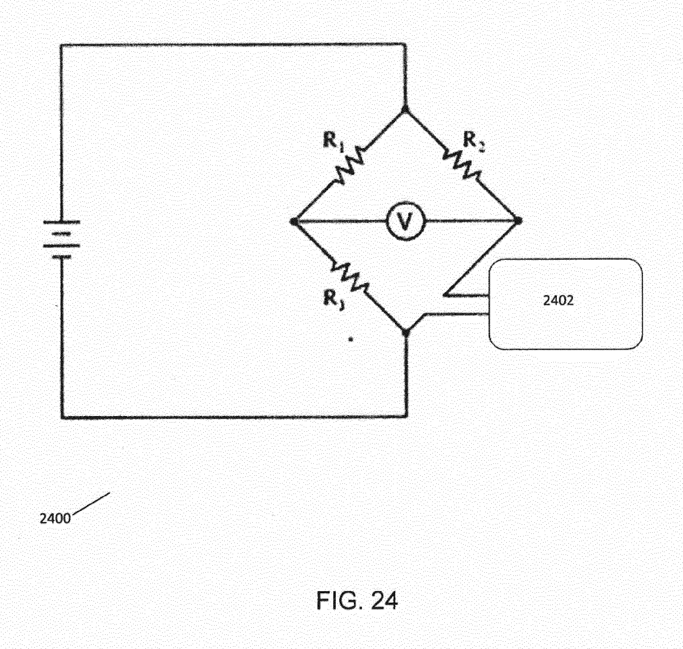

FIG.24 shows a simplified exemplary representative circuit diagram for implementing a piezo-based sensing device **2400** disclosed herein, in an example embodiment. V represents a voltmeter, and R₁, R₂ and R₃ represent a plurality of electrical resistors. Component **2402** represents a piezo-based material (e.g. piezoelectric or piezoresistive) which can be depicted as one or more electric resistors, one or more of which being variable resistors with resistance being dependent on the force applied thereto. It will be appreciated by a skilled person that the position of Component **2402** can be interchanged with any one of R₁, R₂ and R₃ or vice versa if desired. R₁, R₂, R₃ and Component **2402** are connected in a Wheatstone bridge configuration. The bridge configuration shown in FIG. 24 is in a quarter-bridge configuration. Nevertheless, if desired, the bridge can also be operated in a half or full form, that is, with one or more components similar to Component **2402** replacing R₂ or R₁, R₂ and R₃ respectively. One or more fixed or variable electrical resistors can also be added as "dummy" force gauges to complete the bridge circuit as and when desired, for example to negate the effects of temperature changes.

In one example working implementation when the bridge is operated in a quarter configuration shown in FIG. 24, R₃ is set to a value equal to the resistance of Component **2402** with no force applied. R₃ can be a variable resistor to allow ease of setting to zero. The other two resistors R₁ and R₂ are set to be equal to each other. In such an arrangement, when no force applied to Component **2402,** the bridge is symmetrically balanced, that is, the voltmeter V indicates zero volts, representing zero force on the component **2402.** When force is being applied to Component **2402,** its resistance varies, i.e. decreases or increases, respectively, thus unbalancing the bridge and producing a non-zero reading on the voltmeter V. The readings obtained on the voltmeter can then be correlated to the actual mechanical force applied on component **2402.**

The pressure detection assembly may comprise a microelectromechanical system (MEMs). In one embodiment, the pressure detection assembly comprises a piezo-based sensor which measures the force applied to a material by correlating signals based on physical and/or electrical property changes of the material due to mechanical stress. Such material can include but is not limited to crystals, ceramics or semiconductors. The electrical property changes can include but are not limited to changes in conductivity, resistivity, resistance, capacitance and/or generated electric charge of the material. The piezo-based sensor can be selected from a group consisting of a piezoelectric based sensor, a piezoresistive based sensor, a piezocapacitive based sensor or the like. In exemplary embodiments, a force transmitting member is rested on the pressure sensor and transmits the force applied thereto to the pressure sensor without substantial displacement or deformation of the force transmitting member.

Advantageously, the pressure sensor can be easily installed in the optical measurement device without adversely compromising on the overall compactness. Thus, allowing the size of the optical measurement device to be kept at minimum, for example less than about 1 cm in thickness. In such embodiments, little or substantially no displacement or deformation may be required to produce an accurate reading of the applied force. This again beneficially reduces the amount of space within the measurement device required for allowing any displacement or deformation to take place. Even more advantageously, as the moving parts involved are reduced, there may be less wear and tear of the internal components, thereby increasing the life span of the device.

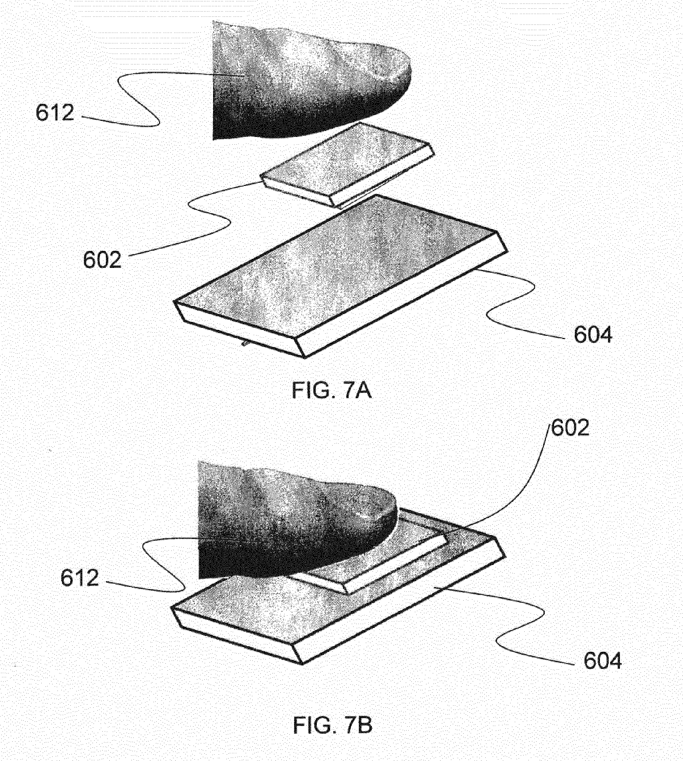

As illustrated in the exploded view of the optical measurement device in FIG. 6A, the pressure sensor **604** may be positioned below the PPG sensor 602, so that the force applied by a user's finger **612** is translated through the PPG sensor **602** to the pressure sensor **604.** The pressure sensor **604** then gathers and tracks the external force exerted by the user's finger **612.** FIG. 7B illustrates an assembled view of the pressure sensor **604** together with the PPG sensor **602** in operation, where the user's finger **606** is placed in contact with the PPG sensor **602.**

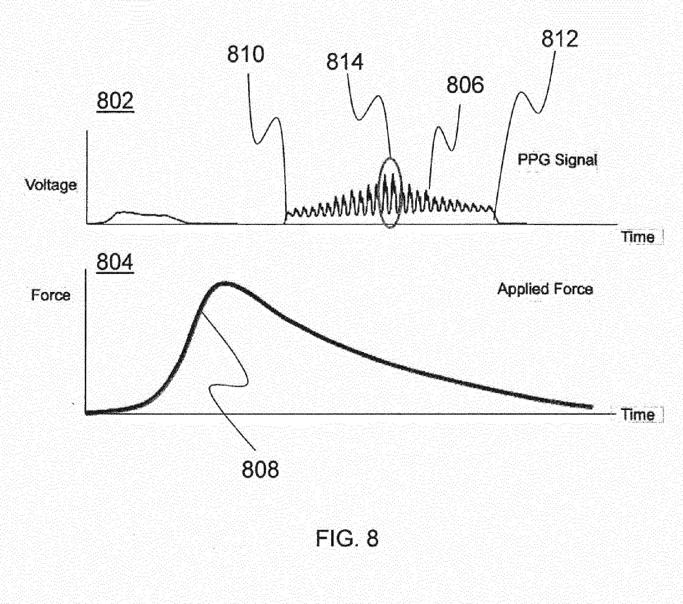

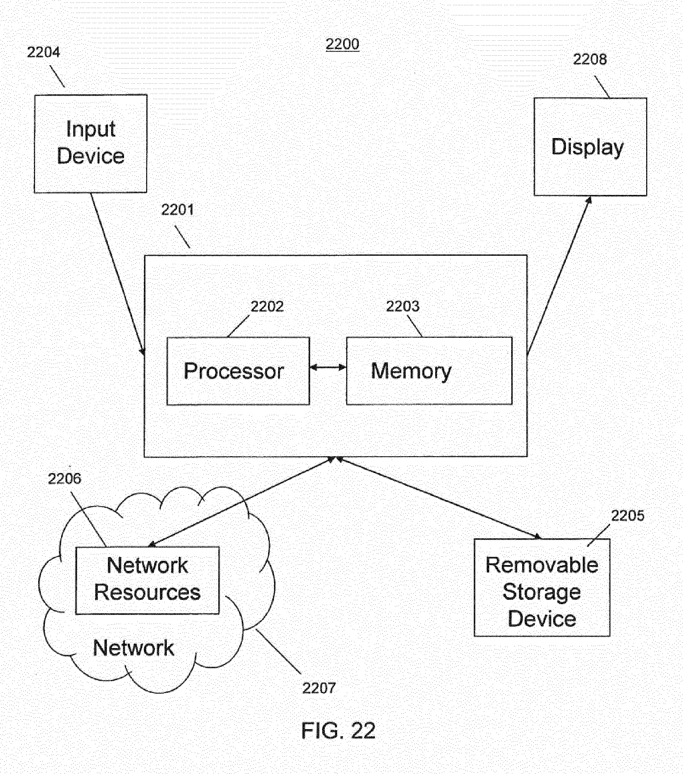

The feedback unit **606** may be a personal mobile processing device. The feedback unit 606 may be a computer including a processor, a memory and optionally a display, as is further described below with regard to FIG. 22. The feedback unit **606** receives a PPG signal and pressure measurements from the optical measurement device **600,** and temporally correlates the PPG signal with the pressure measurements in order to determine an optimal amount of pressure that provides an optimal PPG signal, as shown in the comparison PPG signal graph **802** and applied pressure graph **804,** illustrated in FIG. 8 and described in more detail below.

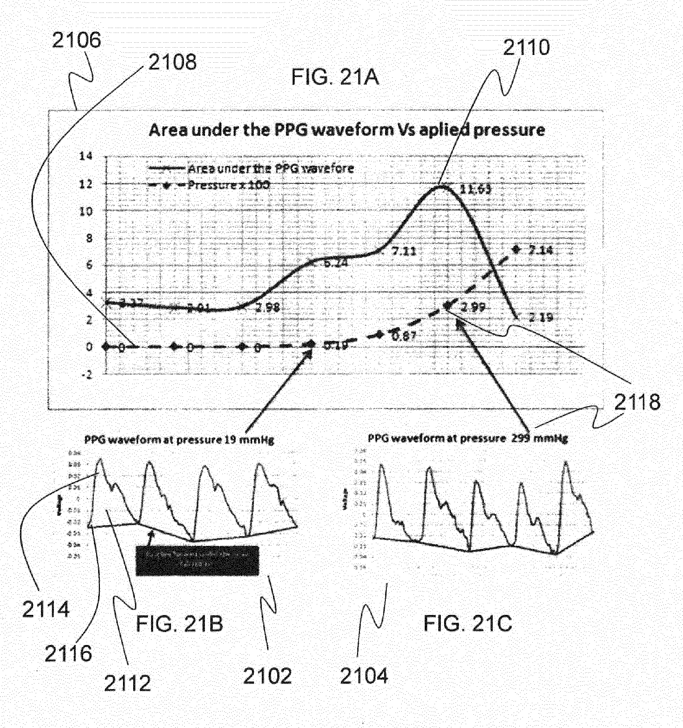

The feedback unit **606** may be provided with a display 614, as illustrated in FIGs. 9A and 9B. The display **614** may provide visual feedback to the user in the form of a graphical user interface (GUI) during the process of measuring the PPG signal. The visual feedback may be a real-time display of the detected PPG signal **616** so that the user can instantly see the effect of varying the amount of pressure being applied to the optical measurement device and adjust the amount of pressure until an optimum PPG signal is displayed. The display **614** may also provide a real-time graphical indication **618** of the pressure being applied. The graphical display **618** of the applied pressure may track the PPG signal **616** on the same graphical display (see Fig. 21A, below), or perhaps be displayed in the form of a vertical pressure status bar **620** positioned on one side of the displayed PPG signal, as illustrated in FIGs. 9A and 9B. The status bar **620** moves up and down depending on the amount of force being applied by the user. In this embodiment, the user identifies an optimal PPG signal in order to determine whether the displayed real-time PPG signal **616** can be improved. However, by displaying the detected PPG signal **616** and possibly the pressure status bar **620,** the feedback unit **606** is not required to compute an amount of pressure that provides an optimum PPG signal, as the user is performing this step manually by analyzing the displayed PPG signal **616** and making adjustments without guidance by the device. FIG. 9B illustrates the feedback unit **606** and the optical measurement device **600** in operation, where a user's finger **612** is positioned on the optical measurement device **600.**

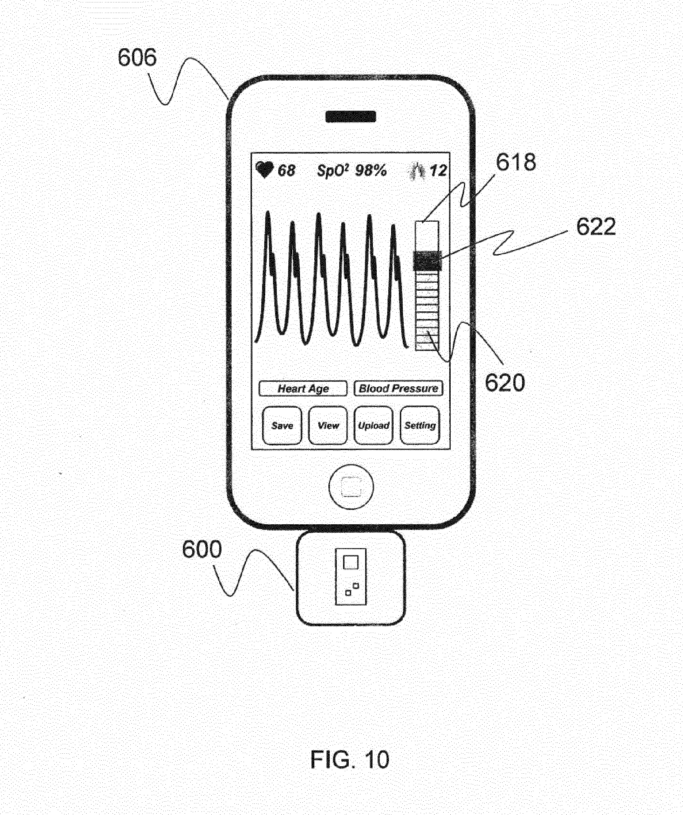

In an exemplary embodiment illustrated in FIG. 10, the feedback unit **606** may generate and display a GUI with a more simplified indication of whether the user should adjust the amount of pressure to provide more, less or the same amount. There may be any number of ways to provide this type of GUI. For example, symbols or shapes - perhaps even color-coded in a traffic-light colored display - may be displayed to tell the user to adjust the amount of force being applied. Similarly, the GUI may simply display words telling the user to "Apply More Pressure, "Apply Less Pressure," or "Apply the Same Amount of Pressure." In FIG. 10, a highlighted box **622** may be placed over the pressure status bar **620** to identify an optimum range at which pressure should be applied for a particular user. In this embodiment, the feedback unit **606** analyzes and compares the measured PPG signal and corresponding applied pressures in real-time in order to determine a range of applied pressure which provides the highest amplitude of PPG signal usually a state of zero transmural pressure. The feedback unit **606** then provides corresponding indicators to the user on the display **614** depending on whether the user is applying pressure within, above or below the determined range.

In an exemplary embodiment, the feedback unit **606** may not require a display, as it could provide audible commands to the user through a speaker or other audio output component. For example, the audio device could simply talk to the user to say "Apply More Pressure," "Apply Less Pressure," or "Apply the Same Amount of Pressure." The audio feedback could also be in the form of musical tones of different pitches or sounds - such as a ringing sound or buzzer sound - which are widely known as positive or negative sounds.

In another exemplary embodiment, the optical measurement device 600 may ask the user to calibrate the device before actual measurement of the PPG signal is carried out. This may involve asking the user to apply a variety of different pressures to the device during a fixed period of time, during which the feedback unit measures the PPG signal detected during that time period and determines a range of applied pressure which obtains an optimal PPG signal. For example, the user may be asked to exert pressure while following a profile of pressure ranges over a period of time, such as the force profile **808** in the applied pressure graph **804** in FIG. 8. As a result of the calibration, the device **600** is able to obtain a range of applied pressure for each individual user, rather than a generalized range which will not be accurate depending on the individual user being measured.

In one exemplary embodiment, the feedback unit **606** may be a portable device, more preferably a personal mobile processing device such as a mobile phone, smartphone, personal digital assistant (PDA), mobile music player, tablet, netbook or laptop, although this list is not exhaustive by any means. However, the feedback unit **606** may not need to be portable, and could similarly be a computer or server. The feedback unit **606** may be connected with the optical detection device **600** in a wired or wireless fashion, more preferably in a cableless configuration via a coupling member of the feedback unit 606, or through a proprietary connector, such a universal serial bus (USB) port or the 30 pin connection used in the Apple® iPhone® (Apple Computer, Inc., Cupertino, CA). In the description herein, cableless configuration is taken to include a connection that is without the use of wires or cables extending from the personal mobile processing device to the optical measurement device.

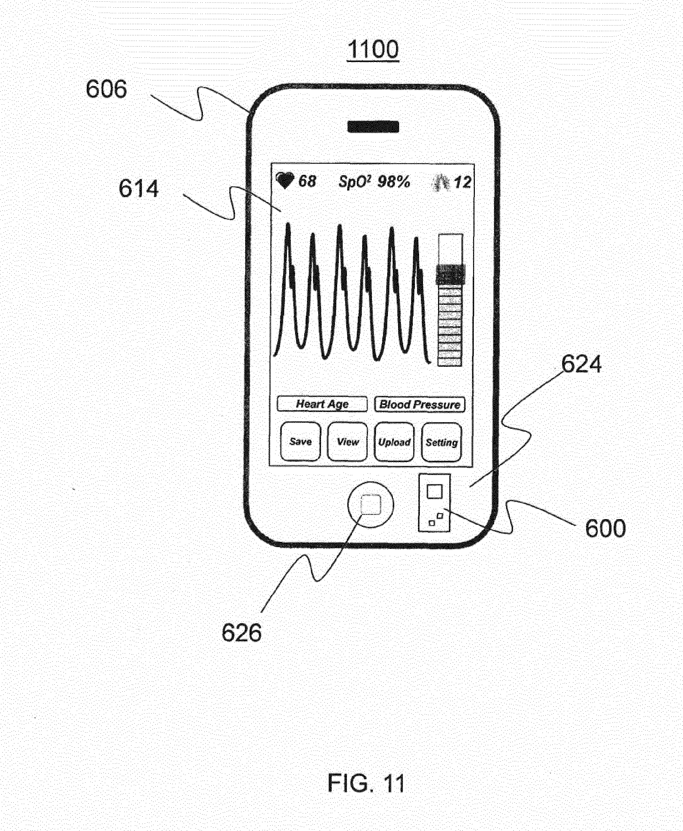

In another embodiment, the portable device may be integrated with the optical detection device as a single optical measurement device **1100,** as shown in FIG. 11. The optical detection device **600** is incorporated within a housing **624** of a portable device **606;** in this case located near a menu button **626** of the portable device **606** and separate from a display **614.** With such a configuration, the portable device **606** is capable of carrying out processing functions for the optical detection device **600,** such as signal conditioning and signal processing. As described below with regard to the block diagram in FIG. 17, the optical detection device **600** integrated with the portable device **606** comprises a sensing portion **628,** while a processing portion **630** would be provided by hardware and firmware of the portable device **606.** The sensing portion **628** preferably includes the illumination and detection assembly **602** and the pressure detection assembly **604,** as illustrated in FIG. 17.

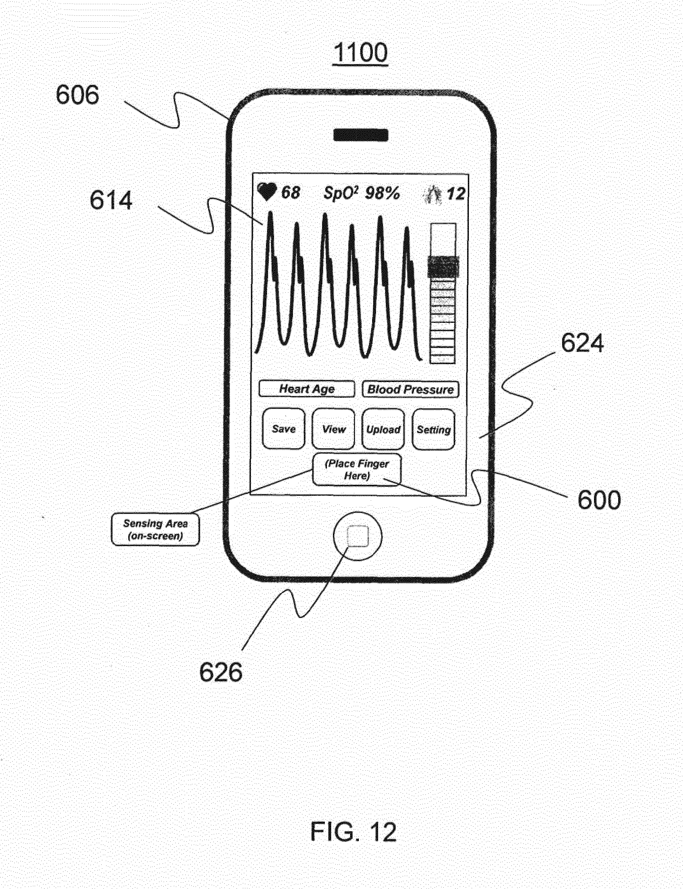

In another exemplary embodiment illustrated in FIG. 12, the optical detection device **600** may be integrated with the display **614** when the display is a touch screen display. Two openings may be created in the touch screen display to permit the transmission of light from the LED light sources and to the photodetector. The components of the touch screen display **614** would have accurate pressure-sensing capabilities to detect the amount of pressure applied on the touch screen display **614,** such that the functions of the pressure detection assembly can be provided by the touch screen display **614** directly, thereby eliminating the need for a separate pressure detection assembly. Therefore, only the illumination and detection assembly of the optical detection device is separately provided, such as red and infrared (IR) LED light sources and a photodetector.

In another exemplary embodiment, the illumination and detection assembly may comprise a camera and flash of a smartphone or other portable device, such that the camera functions as the photodetector while the flash functions as the light source. The flash and camera are located proximate to each other on the portable device, and the flash can be configured with a red LED and infrared LED to output the required wavelengths of light. In this exemplary embodiment, the pressure detection assembly is the only significant modification on the portable device.

Thus, in the exemplary embodiment, an accessory can be provided to a personal mobile processing device. The accessory comprises the pressure detection assembly whereby the pressure assembly is configured to detect an amount of pressure applied, to an illumination and detection assembly of the personal mobile processing device, by a surface portion of a user for measurement. Such an accessory can be removably attached to a casing for the personal mobile processing device. Alternatively, the accessory can be integrally attached to a casing for the personal mobile processing device.

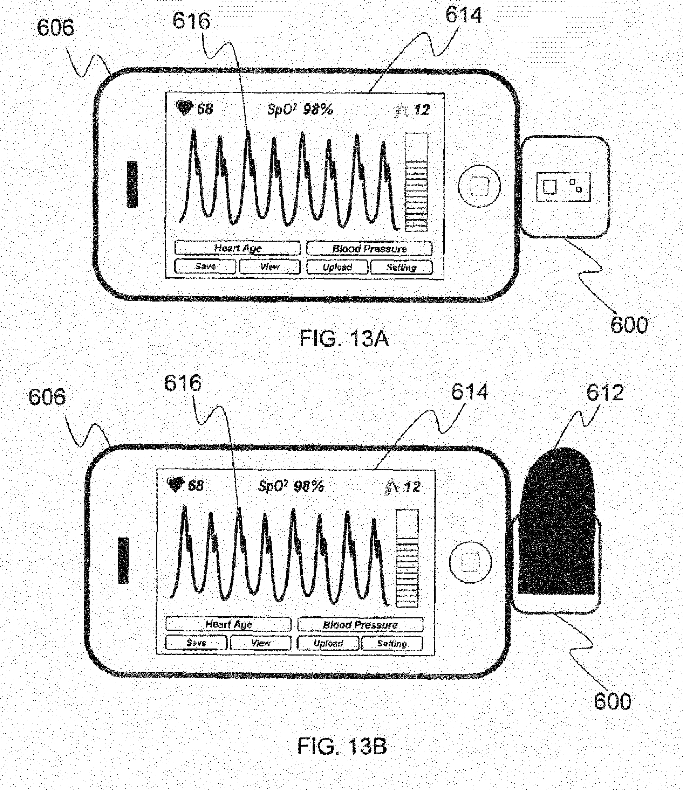

FIGs. 13A and 13B illustrate yet another exemplary embodiment, where the portable device **606** may be oriented in a landscape configuration such that the user views the display **614** horizontally and interacts with the optical detection device **600** in a way that is easier for the user to hold the portable device **606** in the user's hands. In landscape orientation, the user can place a finger **612** on the optical detection device **600** and more easily view a larger time period of the PPG signal **616.**

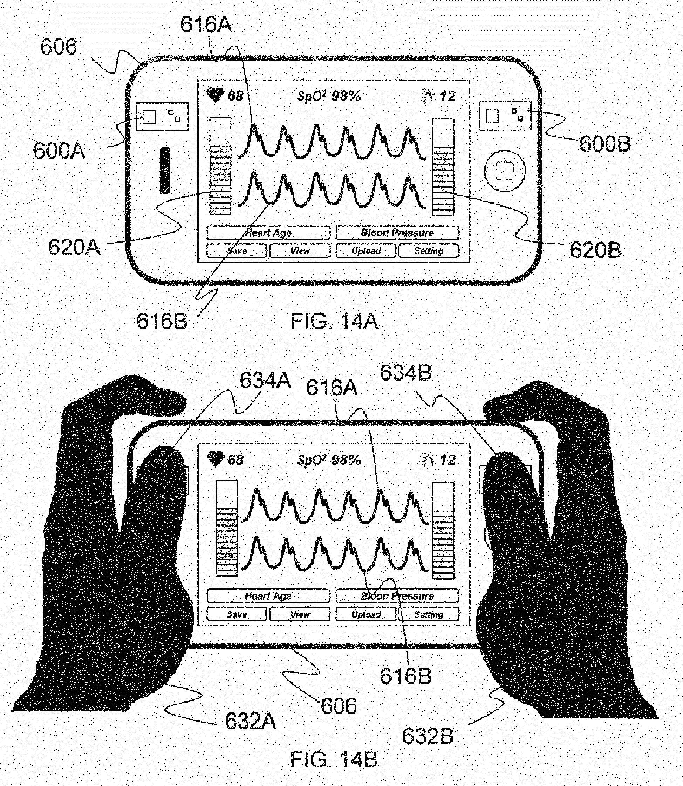

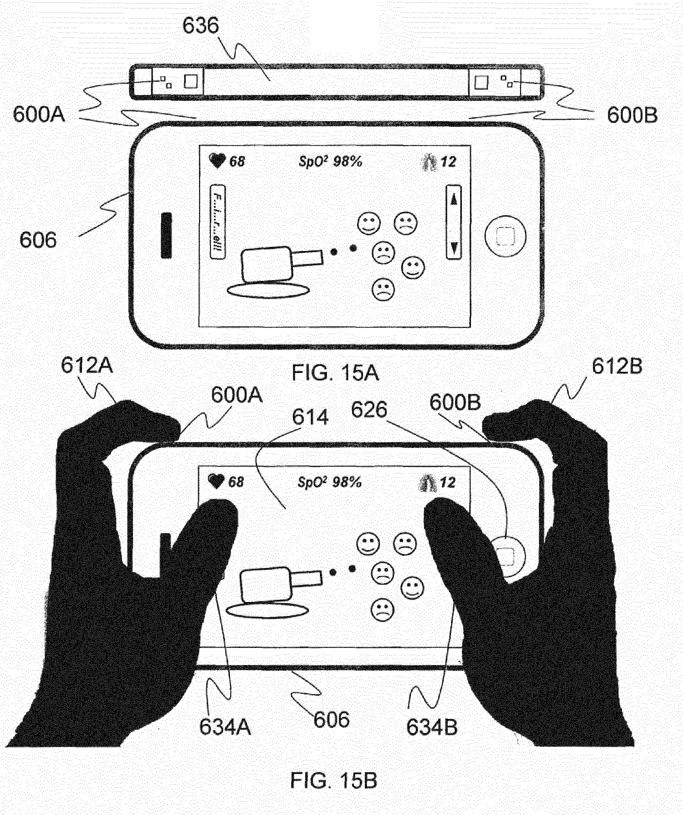

FIGs. 14A and 14B illustrate another exemplary embodiment, where a plurality of optical detection devices **600A** and **600B** are integrated with the portable device **606** for interaction with the user in a landscape orientation. The use of more than one optical detection device will allow measurement of additional physiological properties, As shown in FIG. 14B, the user can easily hold the portable device **606** with both hands **632A** and **632B** while also placing their thumbs **634A** and **634B** on the corresponding optical detection devices **600A** and **600B.** In a similar exemplary embodiment illustrated in FIGs. 15A and 15B, the optical detection devices **600A** and **600B** may be located on a side portion **636** of the portable device **606,** so that the user can place index fingers **612A** and **612B** in contact with the corresponding optical detection devices **600A** and **600B** in a natural configuration. In this embodiment, the user's thumbs **634A** and **634B** are then free to operate the portable device by interacting with the touch screen display **614** or menu button **626** while the index fingers are being sensed by the optical detection devices **600A** and **600B.** In the embodiments illustrated in FIGs. 14 and 15, because there are a plurality of optical detection devices **600A** and **600B,** there may also be a corresponding plurality of PPG signals **616A** and **616B** and pressure status bars **620A** and **620B** accordingly.

The feedback unit may also include software or other computer programmable instructions which carry out instructions relating to receiving and processing the PPG signal, the pressure measurements, and creation of the output to the user relating to the correlation of the detected PPG signal and pressure measurements.

The monitoring of (i) the PPG signal from the illumination and detection assembly and (ii) the amount of force exerted by an individual from the pressure assembly thus enables the optical measurement device to obtain an optimum PPG signal with a high signal to noise ratio. The signal to noise ratio is augmented in an optical signal. The optical measurement device provides for a PPG signal to be acquired at a zero transmural pressure that is unique to each user using the device.

The resulting optimal PPG signal provides a highly accurate measurement of various physiological parameters detected by photoplethysmography, such as a saturation level of oxygen in blood.

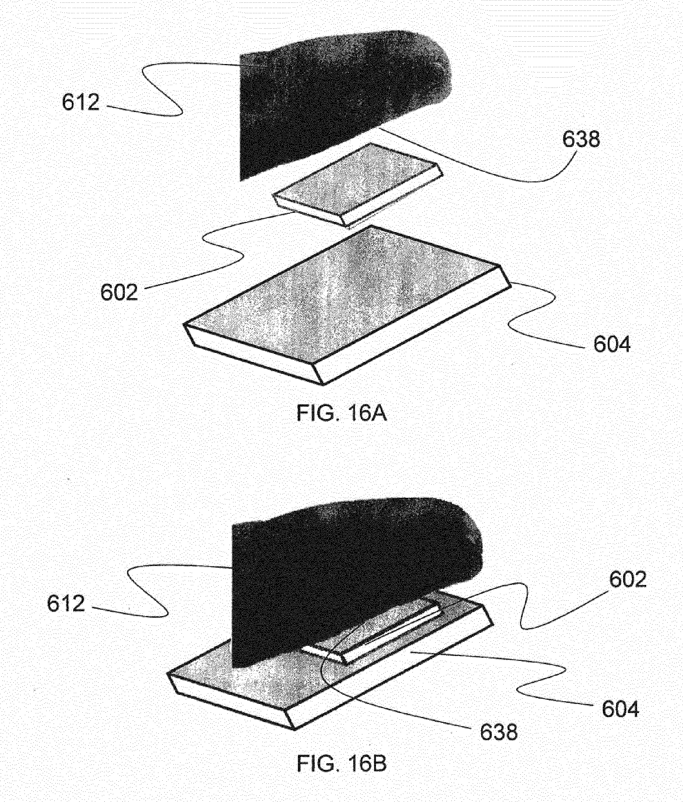

In another embodiment, the optical measurement device further includes acquisition of systolic and diastolic blood pressure parameters. One option for detecting the parameters to determine blood pressure involves placing the side **638** of the finger **612** where the digital artery lies onto the illumination and detection assembly **602,** as illustrated in FIGs.16A and 16B. As shown in FIG. 8, a PPG signal **806** in the PPG signal graph **802** is monitored while the user applies vertical downward force onto the pressure sensor **604** following a pre-determined applied force profile **808** with respect to time, as shown in the applied pressure graph **804.** The basic fundamental behind this analysis is to identify when the PPG signal **806** begins to display a PPG waveform (point **810**) and when the PPG signal finally dies off (point **812**), as these points are indirectly associated with the highest and lowest point of the blood pressure. In addition, with this analysis, the external pressure needed to achieve zero transmural pressure can be determined. When zero transmural pressure is achieved, the PPG waveform reflects the highest amplitude, as shown at area **814** in the PPG signal graph **802.** In FIG. 8, as the amount of applied pressure follows the profile **808** of rapid increase and gradual decrease over time, the PPG waveform **806** changes in amplitude accordingly. Thus, looking at the entire range of PPG waveform from **810** to **812** with respect to applied force **808,** the highest amplitude PPG waveform **814** provides an indication of the corresponding position on the applied pressure graph **804** where an amount of applied pressure results in zero transmural pressure state.

### II. System Architecture

One exemplary embodiment of the optical measurement device is described in further detail below, including its components and their relationships. In the exemplary embodiment below, the optical measurement device is a portable unit coupled to a commercially available feedback unit, corresponding interface, processing and display. That is, the optical measurement device can be provided as an accessory to a personal mobile processing device such as an Apple® iPhone®, although one of skill in the art will recognize that other portable devices may be used.

### A. Interaction Between Illumination/Detection Assembly and the Feedback Unit

The illumination and detection assembly 602 may be connected in a cableless configuration with the feedback unit **606,** in this case a portable device or a personal mobile processing device such as an iPhone®, using the 30 pin connector at the base of the feedback unit **606.** After establishing physical connection of the illumination and detection assembly **602** with the feedback unit **606** or any other form of processing device, a microcontroller unit (MCU) **640** (see FIG. 17) in the illumination and detection assembly 602 extracts information for authentication purposes prior to sending of data to the feedback unit 606 or any other form of processing device. This authentication process may be specific to the iPhone®, as Apple® requires that any device using the 30 pin connector purchase an authentication token from Apple®. Therefore, it will be appreciated that the authentication and MCU 640 can be optional in alternative embodiments.

With the example of an iPhone®, communication is enabled via the Universal Asynchronous Receiver/Transmitter (UART) protocol from the 30 pin connector of the iPhone®. Strings of data are sent to UART every 8 milliseconds from the MCU of the illumination and detection assembly **602** to the iPhone®.

The data is comprised of 2 bytes of header and 10 bytes of payload. The payload is sub-divided into 5 parts, each comprising 2 bytes of data: DC1 (IR), DC2 (Red), PPG1 (IR), PPG2 (Red) and FS (Force Sensor). This data is obtained in a HEX file format and is then converted to back to voltage (V).

Referring back to FIG. 1, DC1 and DC2 provide information for the DC component 104 of the PPG waveform, thus enabling calculation for saturation of peripheral oxygen, or SpO₂.PPG1 and PPG2 establish the actual PPG waveform and provide information for the AC component 102 of the PPG waveform. FS sets out to provide information of the amount of pressure applied to the illumination and detection assembly 602. An example of the data decoding format is show in Table 1, below.

**Table 1: Data Decoding Format**

| Time (ms) | Data from Device | DC1 (Hex) | DC2 (Hex) | PPG1 (Hex) | PPG2 (Hex) | FS (Hex) | DC1 (V) | DC2 (V) | PPG1 (V) | PPG2 (V) | FS (V) |
|---|---|---|---|---|---|---|---|---|---|---|---|
| 0 | F0E24A01023A01F00350055E | 0701 | 023A | 01F0 | 0350 | 055E | 2.8905 | 0.9189 | 0.7996 | 1.3671 | 2.2150 |
| 8 | F0E20A01023A02A80347056D | 0701 | 023A | 02A8 | 0347 | 056D | 2.8905 | 0.9189 | 1.0962 | 1.3528 | 2.2392 |
| 16 | F0E20A01023A030180287B57A | 0701 | 023A | 0318 | 02B7 | 057A | 2.8905 | 0.9189 | 1.2768 | 1.1204 | 2.2602 |
| 24 | FOE20A01023A0314026DO584 | 0701 | 023A | 0314 | 026D | 0584 | 2.8905 | 0.9189 | 1.2703 | 1.0011 | 2.2763 |
| 32 | F0E20A01023A209E02D0058C | 0701 | 023A | 029E | 02DO | 058C | 2.8905 | 0.9189 | 1.0801 | 1.1607 | 2.2892 |
| 40 | F0E20A01023A01E303550591 | 0701 | 023A | 01E3 | 0355 | 0591 | 2.8905 | 0.9189 | 0.7787 | 1.3751 | 2.2973 |
| 48 | F0E20A01023A012003400592 | 0701 | 023A | 012D | 0340 | 0592 | 2.8905 | 0.9189 | 0.4852 | 1.3413 | 2.2989 |
| 56 | F0E20A01023A00C402AE0591 | 0701 | 023A | 00C4 | 02AE | 0591 | 2.8905 | 0.9189 | 0.3160 | 1.1059 | 2.2973 |
| 64 | F0E20A01023A00D4026E058D | 0701 | D23A | 00D0 | 026E | 058D | 2.8905 | 0.9189 | 0.3353 | 1.0027 | 2.2908 |
| 72 | F0E20A01023A014D02DB0585 | 0701 | 023A | 014D | 02DB | 0585 | 2.8906 | 0.9189 | 0.5368 | 1.1785 | 2.2779 |
| 80 | F0E20A01023A0209035A057B | 0701 | 023A | 0209 | 035A | 057B | 2.8905 | 0.9189 | 0.8399 | 1.3832 | 2.2618 |
| 88 | F0E20A01023A02BC0338056E | 0701 | 023A | 02BC | 0338 | 056E | 2.8905 | 0.9189 | 1.1285 | 1.3284 | 2.2408 |
| 96 | F0E20A01023A031F02A5055F | 0701 | 023A | 031F | 02A5 | 055F | 2.8905 | 0.9189 | 1.2881 | 1.0914 | 2.21 67 |
| 104 | F0E20A01023A030B027005AE | 0701 | 023A | 030B | 0270 | 054 E | 2.8905 | 0.9189 | 1.2558 | 1.0060 | 2.1893 |
| 112 | F0E20A01023A028802E5053B | 0701 | 023A | 0288 | 02E5 | 0538 | 2.8905 | 0.9189 | 1.0447 | 1.1946 | 2.1586 |
| 120 | F0E20A01023A01F00350055E | 0701 | 023A | 01C9 | 035E | 0526 | 2.8905 | 0.9189 | 0.7367 | 1.3896 | 2.1248 |

### B. Signal Conditioning

A raw PPG signal includes DC and AC components, both of which containing information critical for waveform analysis. Signal conditioning is therefore performed in order to obtain the information for further processing at the feedback unit. One embodiment of the signal conditioning process will be described below, and may be carried out by components of the illumination and detection assembly **602** illustrated in the block diagram of FIG. 17.

To determine the DC component of the PPG signal, the raw signal **642** obtained from a photo detector **610** is digitized at ADC1 **644.** The digitized signal is passed on to both buffer (IR) **646** and buffer (Red) **648** accordingly, which can store up to 100 samples each before sending collated data to the processor **650.**

Using the raw samples, a baseline DC component can be determined by the processor **650.** At the processor **650,** the digital values for Vsub (IR) and Vsub (RED) (i.e. the DC components) are calculated. The Vsub signals **652** are subsequently converted by a digital-to-analog converter (DAC) **654.**

The determined DC component (Vsub) is then subtracted from the raw signal, Vraw to obtain Vac **656.** The new raw signal, Vac **656,** then undergoes a second stage amplification at a second stage amplifier **658** to obtain Vppg **660,** where the signal to noise ratio is improved compared with Vraw **642.**

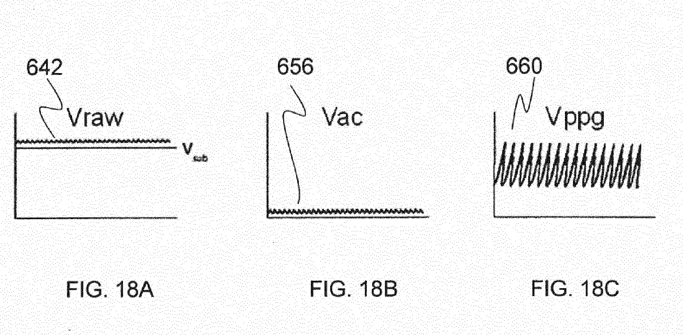

The resolution of the new raw signal **660** is thus enhanced substantially when digitized at ADC2 **662,** as can be seen from the graphical representations of the Vraw signal **642** in FIG. 18A, Vac **656** in FIG. 18B, and Vppg **660** in FIG. 18C.

Referring to FIG. 12, in order to collect the data, an MCU clock **1200** is set to toggle at a predetermined interval to accommodate retrieving results from both LED(IR) **664** and LED(RED) **666** during a respective first interval **1202** and second interval **1204.** In the non-limiting embodiment shown in FIG. 12, the interval **1210** is set to 4milliseconds. The data collection sequence is then repeated in the third interval **1206** and fourth interval **1208.** Before each toggle between the two LEDs, data from ADC1 **644** and ADC2 **662** are taken and sent to UART.

### III. Method of Operation

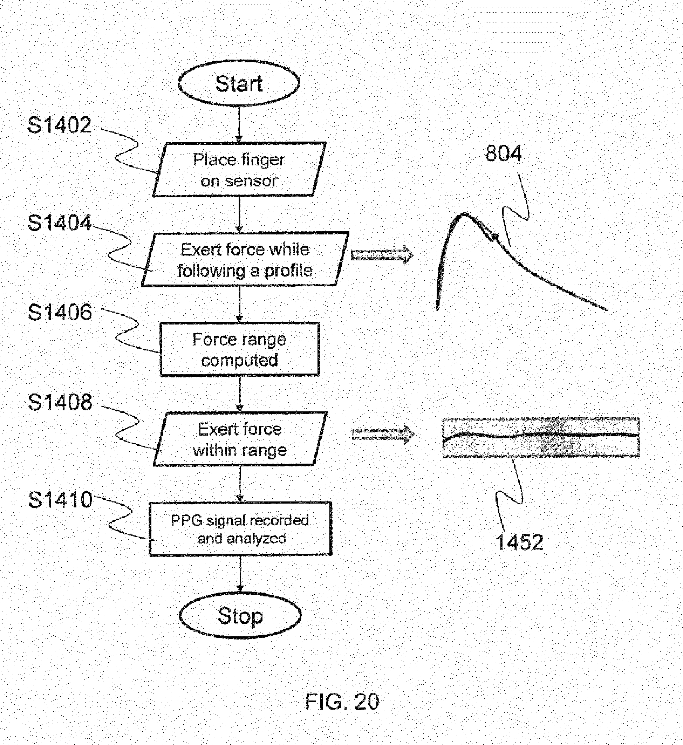

One exemplary embodiment of a method of using the optical measurement device is described herein with reference to FIG. 20, with a corresponding exemplary GUI illustrated in FIGs. 21A - 21C. A user seeking to obtain his or her PPG signals first places a body part, such as a finger, on the sensor/measurement surface of the optical measurement device **(S1402).** That is, a surface portion of the user is placed on the sensor surface for measurement. Calibration of the device to the individual user may be performed **(S1404),** where the user is asked to apply an amount of pressure over a specific period of time, corresponding to a force profile **804,** (see FIG. 8). In other words, the user is asked to vary the applied pressure such that the system can determine an optimum pressure for the user by analyzing the resulting PPG waveforms that result from the variety of applied pressures **(S1406).** The user may also be presented with at least one measured PPG waveform generated by a particular amount of applied pressure, as illustrated in the graphical displays in FIGs. 21B and 21C. FIG.21A is a graphical display **2106** which shows the relationship of a calculated area **2110** under the curve in FIGs. 21B and 21C with respect to applied pressure **2108.** FIGs. 21B and 21C are graphical displays **2102** and **2104,** respectively, which illustrate the different PPG waveforms at different applied pressures, and how the area under curve of the PPG waveform is computed. As shown in FIG. 21A, the optimum pressure **2118** applied in FIG. 21C, 299 mmHg, corresponds to the largest area **2110** of PPG waveform detected during the calibration **(S1404).** Once this optimum pressure is determined, a subsequent measurement period begins, during which the user is asked to apply pressure within an optimum range above and below the optimum pressure **(S1408).** As previously described with regard to FIG. 9A, the amount of pressure being applied by the user may be displayed in a graph **618** on the display **614** so that the user can see the amount of pressure being applied in real-time. The graph **618** may also be displayed using the pressure status bar **620.** If the amount of force being applied by the user falls outside of the optimum range, the system can detect this in real-time and asks the user to increase or decrease the applied pressure in order to remain within the range of optimum pressure and record the best possible PPG signal quality **(S1410).**

Optimum pressure is determined as the pressure at which the measured PPG signal has the largest waveform amplitude, or area **2112** under the PPG waveform, as shown in FIG. 21B by the area **2112** bounded by the PPG signal **2114** and baseline **2116.** FIG. 21A then graphs the variation of the area **2112** under the PPG waveform with respect to the pressure **2108** applied on the sensor. As may be observed in this example, the optimum pressure **2118** is at 299 mmHg, where area **2112** under the curve is at its maximum of 11.63.

FIG. 22 is a block diagram that illustrates an embodiment of a computer/server system **2200** upon which an embodiment of the inventive methodology may be implemented. The system **2200** includes a computer/server platform **2201** including a processor **2202** and memory **2203** which operate to execute instructions, as known to one of skill in the art. The term "computer-readable medium" as used herein refers to any medium that participates in providing instructions to processor **2202** for execution. Additionally, the computer platform **2201** receives input from a plurality of input devices **2204,** such as a keyboard, mouse, touch device or verbal command. The computer platform **2201** may additionally be connected to a removable storage device **2205,** such as a portable hard drive, optical media (CD or DVD), disk media or any other medium from which a computer can read executable code. The computer platform may further be connected to network resources **2206** which connect to the Internet or other components of a local public or private network. The network resources **2206** may provide instructions and data to the computer platform from a remote location on a network **2207.** The connections to the network resources **2206** may be via wireless protocols, such as the 802.11 standards, Bluetooth® or cellular protocols, or via physical transmission media, such as cables or fiber optics. The network resources may include storage devices for storing data and executable instructions at a location separate from the computer platform **2201.** The computer interacts with a display **2208** to output data and other information to a user, as well as to request additional instructions and input from the user. The display **2208** may therefore further act as an input device **2204** for interacting with a user.

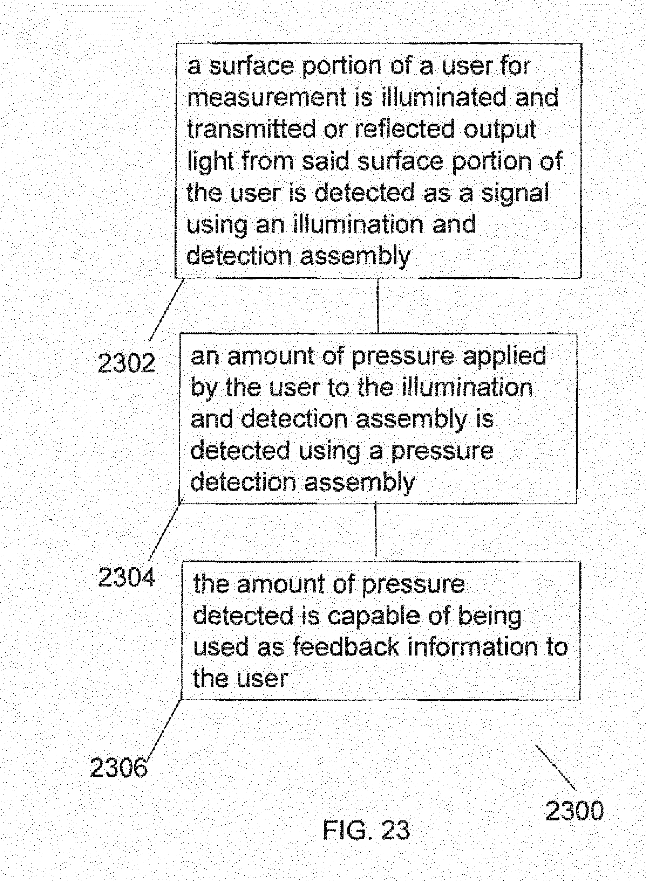

FIG. 23 is a schematic flowchart 2300 illustrating a method for detecting a physiological signal using an optical measurement device in an example embodiment. At step 2302, a surface portion of a user for measurement is illuminated and transmitted or reflected output light from said surface portion of the user is detected as a signal using an illumination and detection assembly. At step 2304, an amount of pressure applied by the user to the illumination and detection assembly is detected using a pressure detection assembly. At step 2306, the amount of pressure detected is capable of being used as feedback information to the user.

## Claims

1. An optical measurement device (2500) comprising:
an illumination and detection assembly (2502, 2504) disposed on a printed circuit board (2512) and comprising a light emitting diode, LED (2502), configured to output light to a surface portion of a user for measurement and a photodetector (2504) configured to detect the output light reflected from said surface portion of the user as a signal, the printed circuit board (2512) further comprising an opening (2520) disposed between the LED (2502) and the photodetector (2504); and
a pressure detection assembly (2506, 2518) configured to detect an amount of pressure applied to the illumination and detection assembly (2502, 2504) by the user, the pressure detection assembly (2506, 2518) comprising a force sensor (2518), the pressure detection assembly (2506, 2518) further comprising a force transmitting member (2506, 2508) including an opaque assembly piece (2506) and an opaque overhead portion (2508), the opaque assembly piece (2506) extending through the opening (2520) in between the LED (2502) and the photodetector (2504), so as to prevent light emitted by the LED (2502) from travelling directly to the photodetector (2504), wherein the opaque overhead portion (2508) includes a first access hole (2508a) for allowing light emitted from the LED (2502) to pass through and a second access hole (2508b) for allowing reflected light to pass through to reach the photodetector (2504),
wherein the illumination and detection assembly (2502, 2504) and the pressure detection assembly (2506, 2518) are configured to communicate with a feedback unit (606) that is configured to correlate a quality of the detected signal with the amount of applied pressure by: computing an area under waveforms of detected signals corresponding to a variety of applied pressure amounts, and determining an optimum pressure to correspond a largest computed area, from the variety of applied pressure amounts.

2. The optical measurement device (2500) as claimed in claim 1, wherein the feedback unit (606) is further configured to provide a feedback related to the optimum pressure to the user.

3. The optical measurement device (2500) as claimed in claim 1 or 2, wherein the illumination and detection assembly (2502, 2504) and pressure detection assembly (2506, 2518) are integrated into a single portable device and the feedback unit (606) is further provided separately.

4. The optical measurement device (2500) as claimed in any one of claims 1 to 3, wherein the pressure assembly (604) comprises non-moveable parts such that the pressure assembly (604) does not substantially deform or displace when in use.

5. The optical measurement device (2500) as claimed in any one of claims 1 to 4, wherein the device is a reflectance-based device.

6. The optical measurement device (2500) as claimed in any one of claims 1 to 5, wherein the illumination and detection assembly (2502, 2504) comprises a red light emitting diode, LED, (2502), an infra-red LED or both.

7. The optical measurement device (2500) as claimed in claim 6, wherein saturation of peripheral oxygen, SPO2, information of the user is derivable from the detected output light information from both the red LED (2502) and the infra-red LED.

8. The optical measurement device (2500) as claimed in any one of claims 1 to 7, further comprising a coupling member (2516) configured for coupling in a cableless configuration to a personal mobile processing device.

9. The optical measurement device (2500) of claim 1, wherein the illumination and detection assembly (2502, 2504), pressure detection assembly (2506, 2518) and feedback unit (606) are integrated into a portable device.

10. The optical measurement device (2500) as claimed in claim 2, wherein the personal mobile processing device comprises the feedback unit (606) configured to correlate the quality of the detected signal with the amount of applied pressure and provide feedback related to the correlation to the user.

11. The optical measurement device (2500) as claimed in any one of claims 2 to 10, wherein the feedback is an indication of whether the user should adjust the amount of pressure being applied to the illumination and detection assembly (2502, 2504).

12. The optical measurement device (2500) as claimed in claim 11, wherein the feedback is configured to display a range of optimal applied pressures along with the actual applied pressure being applied by the user.

13. The optical measurement device (2500) as claimed in claim 12, wherein the range of optimal applied pressures corresponds to a state of zero transmural pressure.

14. The optical measurement device (2500) as claimed in any one of claims 1 to 13, wherein the feedback is a request to the user to increase, decrease or maintain the applied pressure.

15. The optical measurement device (2500) as claimed in any one of claims 1 to 14, wherein the feedback is a real-time visual output of the detected signal and detected applied pressure.

16. The optical measurement device (2500) as claimed in claim 9, wherein the feedback unit (606) is a portable computer including a processor, memory and a display.

17. The optical measurement device (2500) as claimed in any one of claim 9 or 16, wherein the portable device is configured with a plurality of illumination and detection assemblies and a plurality of pressure assemblies.

18. The optical measurement device (600, 2500) as claimed in any one of claims 3 to 17, wherein the illumination and detection assembly (2502, 2504) and the pressure detection assembly (2506, 2518) are configured to communicate with the feedback unit over a wireless network.

19. The optical measurement device (2500) as claimed in any one of claims 1 to 18, wherein the detected signal is a photoplethysmography, PPG, signal.

20. The optical measurement device (2500) as claimed in any one of the preceding claims when dependent on claim 7, wherein the personal mobile processing device is one selected from a group consisting of a mobile phone, a smartphone, a personal digital assistant, PDA, a mobile music player, a tablet computer, a netbook and a laptop.

21. A method for detecting a physiological signal using an optical measurement device (2500), comprising:
illuminating (2302), by a light emitting diode, LED (2502), a surface portion of a user for measurement and detecting, by a photodetector (2504), reflected output light from said surface portion of the user as a signal using an illumination and detection assembly (2502, 2504) disposed on a printed circuit board (2512) comprising an opening (2520) disposed between the LED (2502) and the photodetector (2504);
detecting (2304) an amount of pressure applied by the user to the illumination and detection assembly (2502, 2504) using a pressure detection assembly (2506, 2518) comprising a force sensor (2518), the pressure detection assembly (2506, 2518) further comprising a force transmitting member (2506, 2508) including an opaque assembly piece (2506) and an opaque overhead portion (2508), the opaque assembly piece (2506) extending through the opening (2520) in between the LED (2502) and the photodetector (2504), so as to prevent light emitted by the LED (2502) from travelling directly to the photodetector (2504), wherein the opaque overhead portion (2508) includes a first access hole (2508a) for allowing light emitted from the LED (2502) to pass through and a second access hole (2508b) for allowing reflected light to pass through to reach the photodetector (2504); and
correlating a quality of the detected signal with the amount of applied pressure by:
computing an area under waveforms of detected signals corresponding to a variety of applied pressure amounts, and determining an optimum pressure to correspond a largest computed area, from the variety of applied pressure amounts.

22. The method of claim 21, further comprising providing (2306) feedback related to the optimum pressure to the user using a feedback unit (606).

23. The method as claimed in claim 22, wherein the illumination and detection assembly (2502, 2504) and pressure detection assembly (2506, 2518) are integrated into a single portable device and the feedback unit is further provided separately.

24. The method as claimed in any one of claims 21 to 23, wherein the pressure assembly (604) does not substantially deform or displace when in use.

25. The method as claimed in any one of claims 21 to 24, wherein the measurement device (2500) is a reflectance-based device.

26. The method as claimed in any one of claims 21 to 25, wherein the illumination and detection assembly (2502, 2504) comprises a red light emitting diode, LED (2502), an infra-red LED or both.

27. The method as claimed in claim 26, further comprising deriving saturation of peripheral oxygen, SPO2, information of the user from the detected output light information from both the red LED (2502) and the infra-red LED.

28. The method as claimed in any one of claims 21 to 27, further comprising coupling a coupling member (2516) in a cableless configuration to a personal mobile processing device.

29. The method as claimed in claim 22, further comprising providing an indication to the user of whether the amount of pressure being applied to the illumination and detection assembly (2502, 2504) should be adjusted.

30. The method as claimed in any one of claims 21 to 29, further comprising displaying a range of optimal applied pressures along with the actual applied pressure being applied by the user.

31. The method as claimed in any one of claims 21 to 29, further comprising displaying a range of optimal applied pressures along which corresponds to a state of zero transmural pressure.

32. The method as claimed in any one of claims 21 to 31, further comprising requesting the user to increase, decrease or maintain the applied pressure.

33. The method as claimed in any one of claims 20 to 32, further comprising displaying a real-time visual output of the detected signal and the detected applied pressure.

34. The method as claimed in any one of claims 22 to 33, wherein the feedback unit is a portable computer including a processor, memory and a display.

35. The method as claimed in any one of claims 22 to 34, wherein the illumination and detection assembly (2502, 2504), pressure detection assembly (2506, 2518) and feedback unit (606) are integrated into a portable device.

36. The method as claimed in claim 35, wherein the portable device is configured with a plurality of illumination and detection assemblies and a plurality of pressure assemblies.

37. The method as claimed in claim 34, further comprising wirelessly communicating between the feedback unit (606) and the illumination and detection assembly (2502, 2504) and pressure detection assembly (2506, 2518).

38. The method as claimed in claim 22, wherein the personal mobile processing device comprises the feedback unit.

39. The method as claimed in any one of claims 21 to 38, wherein the detected signal is a photoplethysmography, PPG, signal.

## Patentansprüche

1. Eine optische Messvorrichtung (2500), die folgende Merkmale aufweist:
eine Beleuchtungs- und Erfassungsanordnung (2502, 2504), die auf einer gedruckten Schaltungsplatine (2512) angeordnet ist und eine lichtemittierende Diode, LED (2502), die dazu konfiguriert ist, Licht an einen Oberflächenabschnitt eines Benutzers zur Messung auszugeben, und einen Photodetektor (2504) aufweist, der dazu konfiguriert ist, das Ausgangslicht, das von dem Oberflächenabschnitt des Benutzers reflektiert wird, als Signal zu erfassen, wobei die gedruckte Schaltungsplatine (2512) ferner eine Öffnung (2520) aufweist, die zwischen der LED (2502) und dem Photodetektor (2504) angeordnet ist; und
eine Druckerfassungsanordnung (2506, 2518), die dazu konfiguriert ist, einen Druckbetrag zu erfassen, der durch den Benutzer auf die Beleuchtungs- und Erfassungsanordnung (2502, 2504) ausgeübt wird, wobei die Druckerfassungsanordnung (2506, 2518) einen Kraftsensor (2518) aufweist, wobei die Druckerfassungsanordnung (2506, 2518) ferner ein Kraftübertragungsbauglied (2506, 2508) aufweist, das ein lichtundurchlässiges Anordnungsstück (2506) und ein lichtundurchlässiges Überkopfstück (2508) aufweist, wobei das lichtundurchlässige Anordnungsstück (2506) sich durch die Öffnung (2520) zwischen der LED (2502) und dem Photodetektor (2504) erstreckt, um zu verhindern, dass durch die LED (2502) emittiertes Licht sich direkt zu dem Photodetektor (2504) bewegt, wobei der lichtundurchlässige Überkopfabschnitt (2508) ein erstes Zugangsloch (2508a), durch das von der LED (2502) emittiertes Licht hindurchtreten kann, und ein zweites Zugangsloch (2508b) umfasst, durch das reflektiertes Licht hindurchtreten kann, um den Photodetektor (2504) zu erreichen,
wobei die Beleuchtungs- und Erfassungsanordnung (2502, 2504) und die Druckerfassungsanordnung (2506, 2518) dazu konfiguriert sind, mit einer Feedbackeinheit (606) zu kommunizieren, die dazu konfiguriert ist, eine Qualität des erfassten Signals mit dem Betrag an ausgeübtem Druck zu korrelieren, durch: Berechnen einer Fläche unter Wellenformen von erfassten Signalen, die einer Vielzahl von ausgeübten Druckbeträgen entsprechen, und Bestimmen eines optimalen Drucks, der einer größten berechneten Fläche entsprechen soll, aus der Vielzahl von ausgeübten Druckbeträgen.

2. Die optische Messvorrichtung (2500) gemäß Anspruch 1, wobei die Feedbackeinheit (606) ferner dazu konfiguriert ist, dem Benutzer ein Feedback, das sich auf den optimalen Druck bezieht, bereitzustellen.

3. Die optische Messvorrichtung (2500) gemäß Anspruch 1 oder 2, wobei die Beleuchtungs- und Erfassungsanordnung (2502, 2504) und die Druckerfassungsanordnung (2506, 2518) in einer einzigen tragbaren Vorrichtung integriert sind und die Feedbackeinheit (606) ferner getrennt bereitgestellt ist.

4. Die optische Messvorrichtung (2500) gemäß einem der Ansprüche 1 bis 3, wobei die Druckanordnung (604) nicht bewegbare Teile derart aufweist, dass die Druckanordnung (604) sich bei Gebrauch nicht wesentlich verformt oder verschiebt.

5. Die optische Messvorrichtung (2500) gemäß einem der Ansprüche 1 bis 4, wobei die Vorrichtung eine Vorrichtung auf Basis des Reflexionsvermögens ist.

6. Die optische Messvorrichtung (2500) gemäß einem der Ansprüche 1 bis 5, wobei die Beleuchtungs- und Erfassungsanordnung (2502, 2504) eine rote lichtemittierende Diode, LED, (2502), eine infrarote LED oder beides aufweist.

7. Die optische Messvorrichtung (2500) gemäß Anspruch 6, wobei Informationen über die periphere Sauerstoffsättigung, SPO2, des Benutzers aus den erfassten Ausgangslichtinformationen von sowohl der roten LED (2502) als auch der infraroten LED gewonnen werden könne.

8. Die optische Messvorrichtung (2500) gemäß einem der Ansprüche 1 bis 7, die ferner ein Kopplungsbauglied (2516) aufweist, das dazu konfiguriert ist, in einer kabellosen Konfiguration eine Kopplung mit einer persönlichen mobilen Verarbeitungsvorrichtung herzustellen.

9. Die optische Messvorrichtung (2500) gemäß Anspruch 1, wobei die Beleuchtungs- und Erfassungsanordnung (2502, 2504), die Druckerfassungsanordnung (2506, 2518) und die Feedbackeinheit (606) in einer tragbaren Vorrichtung integriert sind.

10. Die optische Messvorrichtung (2500) gemäß Anspruch 2, wobei die persönliche mobile Verarbeitungsvorrichtung die Feedbackeinheit (606) aufweist, die dazu konfiguriert ist, die Qualität des erfassten Signals mit dem Betrag an ausgeübtem Druck zu korrelieren und dem Benutzer ein Feedback, das sich auf die Korrelation bezieht, bereitzustellen.

11. Die optische Messvorrichtung (2500) gemäß einem der Ansprüche 2 bis 10, wobei das Feedback eine Angabe darüber ist, ob der Benutzer den Druckbetrag, der auf die Beleuchtungs- und Erfassungsanordnung (2502, 2504) ausgeübt wird, anpassen sollte.

12. Die optische Messvorrichtung (2500) gemäß Anspruch 11, wobei das Feedback dazu konfiguriert ist, einen Bereich von optimalen ausgeübten Drücken zusammen mit dem tatsächlich ausgeübten Druck, der durch den Benutzer ausgeübt wird, anzuzeigen.

13. Die optische Messvorrichtung (2500) gemäß Anspruch 12, wobei der Bereich von optimalen ausgeübten Drücken einem Zustand eines transmuralen Drucks von null entspricht.

14. Die optische Messvorrichtung (2500) gemäß einem der Ansprüche 1 bis 13, wobei das Feedback eine Aufforderung an den Benutzer ist, den ausgeübten Druck zu erhöhen, zu senken oder beizubehalten.

15. Die optische Messvorrichtung (2500) gemäß einem der Ansprüche 1 bis 14, wobei das Feedback eine visuelle Echtzeitausgabe des erfassten Signals und des erfassten ausgeübten Drucks ist.

16. Die optische Messvorrichtung (2500) gemäß Anspruch 9, wobei die Feedbackeinheit (606) ein tragbarer Computer ist, der einen Prozessor, einen Speicher und eine Anzeige umfasst.

17. Die optische Messvorrichtung (2500) gemäß einem der Ansprüche 9 oder 16, wobei die tragbare Vorrichtung mit einer Mehrzahl von Beleuchtungs- und Erfassungsanordnungen und einer Mehrzahl von Druckanordnungen konfiguriert ist.

18. Die optische Messvorrichtung (600, 2500) gemäß einem der Ansprüche 3 bis 17, wobei die Beleuchtungs- und Erfassungsanordnung (2502, 2504) und die Druckerfassungsanordnung (2506, 2518) dazu konfiguriert sind, mit der Feedbackeinheit über ein drahtloses Netzwerk zu kommunizieren.

19. Die optische Messvorrichtung (2500) gemäß einem der Ansprüche 1 bis 18, wobei das erfasste Signal ein Photoplethysmographie-Signal, PPG-Signal, ist.

20. Die optische Messvorrichtung (2500) gemäß einem der vorhergehenden Ansprüche bei Abhängigkeit von Anspruch 7, wobei die persönliche mobile Verarbeitungsvorrichtung eine Vorrichtung ist, die aus einer Gruppe ausgewählt ist, die aus einem Mobiltelefon, einem Smartphone, einem persönlichen digitalen Assistenten, PDA, einem mobilen Musikspieler, einem Tablet-Computer, einem Netbook und einem Laptop besteht.

21. Ein Verfahren zum Erfassen eines physiologischen Signals unter Verwendung einer optischen Messvorrichtung (2500), das folgende Schritte aufweist:
Beleuchten (2302), mittels einer lichtemittierenden Diode, LED (2502), eines Oberflächenabschnitts eines Benutzers zur Messung und Erfassen, durch einen Photodetektor (2504), von reflektiertem Angangslicht von dem Oberflächenabschnitt des Benutzers als Signal, wobei eine Beleuchtungs- und Erfassungsanordnung (2502, 2504) verwendet wird, die auf einer gedruckten Schaltungsplatine (2512) angeordnet ist, die eine Öffnung (2520) aufweist, die zwischen der LED (2502) und dem Photodetektor (2504) angeordnet ist;
Erfassen (2304) eines Druckbetrags, der durch den Benutzer auf die Beleuchtungs- und Erfassungsanordnung (2502, 2504) ausgeübt wird, wobei eine Druckerfassungsanordnung (2506, 2518) verwendet wird, die einen Kraftsensor (2518) aufweist, wobei die Druckerfassungsanordnung (2506, 2518) ferner ein Kraftübertragungsbauglied (2506, 2508) aufweist, das ein lichtundurchlässiges Anordnungsstück (2506) und einen lichtundurchlässigen Überkopfabschnitt (2508) aufweist, wobei das lichtundurchlässige Anordnungsstück (2506) sich durch die Öffnung (2520) zwischen der LED (2502) und dem Photodetektor (2504) erstreckt, um zu verhindern, dass durch die LED (2502) emittiertes Licht sich direkt zu dem Photodetektor (2504) bewegt, wobei der lichtundurchlässige Überkopfabschnitt (2508) ein erstes Zugangsloch (2508a), durch das von der LED (2502) emittiertes Licht hindurchtreten kann, und ein zweites Zugangsloch (2508b) umfasst, durch das reflektiertes hindurchtreten kann, um den Photodetektor (2504) zu erreichen; und
Korrelieren einer Qualität des erfassten Signals mit dem Betrag an ausgeübtem Druck durch:
Berechnen einer Fläche unter Wellenformen von erfassten Signalen, die einer Vielzahl von ausgeübten Druckbeträgen entsprechen, und Bestimmen eines optimalen Drucks, der einer größten berechneten Fläche entsprechen soll, aus der Vielzahl von ausgeübten Druckbeträgen.

22. Das Verfahren gemäß Anspruch 21, das ferner ein Bereitstellen (2306) von Feedback, das sich auf den optimalen Druck bezieht, für den Benutzer unter Verwendung einer Feedbackeinheit (606) aufweist.

23. Das Verfahren gemäß Anspruch 22, bei dem die Beleuchtungs- und Erfassungsanordnung (2502, 2504) und die Druckerfassungsanordnung (2506, 2518) in einer einzigen tragbaren Vorrichtung integriert sind und die Feedbackeinheit ferner getrennt bereitgestellt wird.

24. Das Verfahren gemäß einem der Ansprüche 21 bis 23, bei dem die Druckanordnung (604) sich bei Gebrauch nicht wesentlich verformt oder verschiebt.

25. Das Verfahren gemäß einem der Ansprüche 21 bis 24, bei dem die Messvorrichtung (2500) eine Vorrichtung auf Basis des Reflexionsvermögens ist.

26. Das Verfahren gemäß einem der Ansprüche 21 bis 25, bei dem die Beleuchtungs- und Erfassungsanordnung (2502, 2504) eine rote lichtemittierende Diode, LED, (2502), eine infrarote LED oder beides aufweist.

27. Das Verfahren gemäß Anspruch 26, das ferner ein Gewinnen von Informationen über die periphere Sauerstoffsättigung, SPO2, des Benutzers aus den erfassten Ausgangslichtinformationen von sowohl der roten LED (2502) als auch der infraroten LED aufweist.

28. Das Verfahren gemäß einem der Ansprüche 21 bis 27, das ferner ein Koppeln eines Kopplungsbauglieds (2516) in einer kabellosen Konfiguration mit einer persönlichen mobilen Verarbeitungsvorrichtung aufweist.

29. Das Verfahren gemäß Anspruch 22, das ferner ein Bereitstellen einer Angabe gegenüber dem Benutzer aufweist, ob der Druckbetrag, der auf die Beleuchtungs- und Erfassungsanordnung (2502, 2504) ausgeübt wird, angepasst werden sollte.

30. Das Verfahren gemäß einem der Ansprüche 21 bis 29, das ferner ein Anzeigen eines Bereichs von optimalen ausgeübten Drücken zusammen mit dem tatsächlich ausgeübten Druck, der durch den Benutzer ausgeübt wird, aufweist.

31. Das Verfahren gemäß einem der Ansprüche 21 bis 29, das ferner ein Anzeigen eines Bereichs von optimalen ausgeübten Drücken aufweist, der einem Zustand eines transmuralen Drucks von null entspricht.

32. Das Verfahren gemäß einem der Ansprüche 21 bis 31, das ferner ein Auffordern des Benutzers aufweist, den ausgeübten Druck zu erhöhen, zu senken oder beizubehalten.

33. Das Verfahren gemäß einem der Ansprüche 20 bis 32, das ferner ein Anzeigen einer visuellen Echtzeitausgabe des erfassten Signals und des erfassten ausgeübten Drucks aufweist.

34. Das Verfahren gemäß einem der Ansprüche 22 bis 33, bei dem die Feedbackeinheit ein tragbarer Computer ist, der einen Prozessor, einen Speicher und eine Anzeige umfasst.

35. Das Verfahren gemäß einem der Ansprüche 22 bis 34, bei dem die Beleuchtungs- und Erfassungsanordnung (2502, 2504), die Druckerfassungsanordnung (2506, 2518) und die Feedbackeinheit (606) in einer tragbaren Vorrichtung integriert werden.

36. Das Verfahren gemäß Anspruch 35, bei dem die tragbare Vorrichtung mit einer Mehrzahl von Beleuchtungs- und Erfassungsanordnungen und einer Mehrzahl von Druckanordnungen konfiguriert ist.

37. Das Verfahren gemäß Anspruch 34, das ferner ein drahtloses Kommunizieren zwischen der Feedbackeinheit (606) und der Beleuchtungs- und Erfassungsanordnung (2502, 2504) und der Druckerfassungsanordnung (2506, 2518) aufweist.

38. Das Verfahren gemäß Anspruch 22, bei dem die persönliche mobile Verarbeitungsvorrichtung die Feedbackeinheit aufweist.

39. Das Verfahren gemäß einem der Ansprüche 21 bis 38, bei dem das erfasste Signal ein Photoplethysmographie-Signal, PPG-Signal, ist.

## Revendications

1. Dispositif de mesure optique (2500), comprenant:
un ensemble d'éclairage et de détection (2502, 2504) disposé sur une carte de circuit imprimé (2512) et comprenant une diode électroluminescente, LED, (2502), configuré pour sortir de la lumière vers une partie de surface d'un utilisateur pour la mesure, et un photo-détecteur (2504) configuré pour détecter la lumière sortie réfléchie par ladite partie de surface de l'utilisateur comme un signal, la carte de circuit imprimé (2512) comprenant par ailleurs une ouverture (2520) disposée entre la LED (2502) et le photo-détecteur (2504); et
un ensemble de détection de pression (2506, 2518) configuré pour détecter une quantité de pression appliquée à l'ensemble d'éclairage et de détection (2502, 2504) par l'utilisateur, l'ensemble de détection de pression (2506, 2518) comprenant un capteur de force (2518), l'ensemble de détection de pression (2506, 2518) comprenant par ailleurs un élément de transmission de force (2506, 2508) comportant une pièce d'assemblage opaque (2506) et une partie supérieure opaque (2508), la pièce d'assemblage opaque (2506) s'étendant à travers l'ouverture (2520) entre la LED (2502) et le photo-détecteur (2504), de manière à empêcher que la lumière émise par la LED (2502) ne se déplace directement vers le photo-détecteur (2504), où la partie supérieure opaque (2508) comporte un premier trou d'accès (2508a) destiné à permettre que passe la lumière émise par la LED (2502) et un deuxième trou d'accès (2508b) destiné à permettre que passe la lumière réfléchie pour atteindre le photo-détecteur (2504),
dans lequel l'ensemble d'éclairage et de détection (2502, 2504) et l'ensemble de détection de pression (2506, 2518) sont configurés pour communiquer avec une unité de rétroaction (606) qui est configurée pour corréler une qualité du signal détecté avec la quantité de pression appliquée: en calculant une zone sous des formes d'onde des signaux détectés correspondant à une variété de quantités de pression appliquées, et en déterminant une pression optimale qui correspond à une zone calculée la plus grande, parmi la variété des quantités de pression appliquées.

2. Dispositif de mesure optique (2500) selon la revendication 1, dans lequel l'unité de rétroaction (606) est par ailleurs configurée pour fournir à l'utilisateur une rétroaction relative à la pression optimale.

3. Dispositif de mesure optique (2500) selon la revendication 1 ou 2, dans lequel l'ensemble d'éclairage et de détection (2502, 2504) et l'ensemble de détection de pression (2506, 2518) sont intégrés dans un seul dispositif portable et l'unité de rétroaction (606) est par ailleurs prévue séparément.

4. Dispositif de mesure optique (2500) selon l'une quelconque des revendications 1 à 3, dans lequel l'ensemble de pression (604) comprend des parties non déplaçables de sorte que l'ensemble de pression (604) ne se déforme ou ne se déplace pas sensiblement lors de son utilisation.

5. Dispositif de mesure optique (2500) selon l'une quelconque des revendications 1 à 4, dans lequel le dispositif est un dispositif à base de réflectance.

6. Dispositif de mesure optique (2500) selon l'une quelconque des revendications 1 à 5, dans lequel l'ensemble d'éclairage et de détection (2502, 2504) comprend une diode électroluminescente rouge, LED, (2502), une LED infrarouge, ou les deux.

7. Dispositif de mesure optique (2500) selon la revendication 6, dans lequel les informations de saturation d'oxygène périphérique, SPO2, de l'utilisateur peuvent être dérivées des informations de lumière sorties détectées tant de la LED rouge (2502) que de la LED infrarouge.

8. Dispositif de mesure optique (2500) selon l'une quelconque des revendications 1 à 7, comprenant par ailleurs un élément de couplage (2516) configuré pour se coupler dans une configuration sans câble à un dispositif de traitement mobile personnel.

9. Dispositif de mesure optique (2500) selon la revendication 1, dans lequel l'ensemble d'éclairage et de détection (2502, 2504), l'ensemble de détection de pression (2506, 2518) et l'unité de rétroaction (606) sont intégrés dans un dispositif portable.

10. Dispositif de mesure optique (2500) selon la revendication 2, dans lequel le dispositif de traitement mobile personnel comprend l'unité de rétroaction (606) configurée pour corréler la qualité du signal détecté avec la quantité de pression appliquée et pour fournir à l'utilisateur une rétroaction relative à la corrélation.

11. Dispositif de mesure optique (2500) selon l'une quelconque des revendications 2 à 10, dans lequel la rétroaction est une indication de si l'utilisateur doit ajuster la quantité de pression appliquée à l'ensemble d'éclairage et de détection (2502, 2504).

12. Dispositif de mesure optique (2500) selon la revendication 11, dans lequel la rétroaction est configurée pour afficher une plage de pressions appliquées optimales ensemble avec la pression appliquée réelle appliquée par l'utilisateur.

13. Dispositif de mesure optique (2500) selon la revendication 12, dans lequel la plage de pressions appliquées optimales correspond à un état de pression transmurale nulle.

14. Dispositif de mesure optique (2500) selon l'une quelconque des revendications 1 à 13, dans lequel la rétroaction est une demande à l'utilisateur d'augmenter, de diminuer ou de maintenir la pression appliquée.

15. Dispositif de mesure optique (2500) selon l'une quelconque des revendications 1 à 14, dans lequel la rétroaction est une sortie visuelle en temps réel du signal détecté et de la pression appliquée détectée.

16. Dispositif de mesure optique (2500) selon la revendication 9, dans lequel l'unité de rétroaction (606) est un ordinateur portable comportant un processeur, une mémoire et un affichage.

17. Dispositif de mesure optique (2500) selon l'une quelconque des revendications 9 ou 16, dans lequel le dispositif portable est configuré avec une pluralité d'ensembles d'éclairage et de détection et une pluralité d'ensembles de pression.

18. Dispositif de mesure optique (600, 2500) selon l'une quelconque des revendications 3 à 17, dans lequel l'ensemble d'éclairage et de détection (2502, 2504) et l'ensemble de détection de pression (2506, 2518) sont configurés pour communiquer avec l'unité de rétroaction par un réseau sans fil.

19. Dispositif de mesure optique (2500) selon l'une quelconque des revendications 1 à 18, dans lequel le signal détecté est un signal de photopléthysmographie, PPG.

20. Dispositif de mesure optique (2500) selon l'une quelconque des revendications précédentes lorsque dépendante de la revendication 7, dans lequel le dispositif de traitement mobile personnel est l'un sélectionné parmi un groupe constitué d'un téléphone mobile, d'un téléphone intelligent, d'un assistant numérique personnel, PDA, d'un reproducteur de musique mobile, d'une tablette, d'un netbook et d'un ordinateur portable.

21. Procédé de détection d'un signal physiologique à l'aide d'un dispositif de mesure optique (2500), comprenant le fait de:
éclairer (2302), par une diode électroluminescente, LED (2502), une partie de surface d'un utilisateur pour mesurer et détecter, par un photo-détecteur (2504), la lumière sortie réfléchie par ladite partie de surface de l'utilisateur comme un signal à l'aide d'un ensemble d'éclairage et de détection (2502, 2504) disposé sur une carte de circuit imprimé (2512) comprenant une ouverture (2520) disposée entre la LED (2502) et le photo-détecteur (2504);
détecter (2304) une quantité de pression appliquée par l'utilisateur à l'ensemble d'éclairage et de détection (2502, 2504) à l'aide d'un ensemble de détection de pression (2506, 2518) comprenant un capteur de force (2518), l'ensemble de détection de pression (2506, 2518) comprenant par ailleurs un élément de transmission de force (2506, 2508) comportant une pièce d'assemblage opaque (2506) et une partie supérieure opaque (2508), la pièce d'assemblage opaque (2506) s'étendant à travers l'ouverture (2520) entre la LED (2502) et le photo-détecteur (2504), de manière à empêcher que la lumière émise par la LED (2502) ne se déplace directement vers le photo-détecteur (2504), dans lequel la partie supérieure opaque (2508) comporte un premier trou d'accès (2508a) pour permettre que passe la lumière émise par la LED (2502) et un deuxième trou d'accès (2508b) pour permettre que passe la lumière réfléchie pour atteindre le photo-détecteur (2504); et
corréler une qualité du signal détecté avec la quantité de pression appliquée:
en calculant une zone sous des formes d'onde des signaux détectés correspondant à une variété de quantités de pression appliquées, et en déterminant une pression optimale qui correspond à une zone calculée la plus grande, parmi la variété des quantités de pression appliquées.

22. Procédé selon la revendication 21, comprenant par ailleurs le fait de fournir (2306) à l'utilisateur une rétroaction relative à la pression optimale à l'aide d'une unité de rétroaction (606).

23. Procédé selon la revendication 22, dans lequel l'ensemble d'éclairage et de détection (2502, 2504) et l'ensemble de détection de pression (2506, 2518) sont intégrés dans un seul dispositif portable et l'unité de rétroaction est par ailleurs prévue séparément.

24. Procédé selon l'une quelconque des revendications 21 à 23, dans lequel l'ensemble de pression (604) ne se déforme ou ne se déplace pas sensiblement lors de son utilisation.

25. Procédé selon l'une quelconque des revendications 21 à 24, dans lequel le dispositif de mesure (2500) est un dispositif à base de réflectance.

26. Procédé selon l'une quelconque des revendications 21 à 25, dans lequel l'ensemble d'éclairage et de détection (2502, 2504) comprend une diode électroluminescente rouge, LED (2502), une LED infrarouge, ou les deux.

27. Procédé selon la revendication 26, comprenant par ailleurs le fait de dériver les informations de saturation d'oxygène périphérique, SP02, de l'utilisateur à partir des informations de lumière sorties détectées tant de la LED rouge (2502) que de la LED infrarouge.

28. Procédé selon l'une quelconque des revendications 21 à 27, comprenant par ailleurs le fait de coupler un élément de couplage (2516) dans une configuration sans câble à un dispositif de traitement mobile personnel.

29. Procédé selon la revendication 22, comprenant par ailleurs le fait de fournir à l'utilisateur une indication de si la quantité de pression appliquée à l'ensemble d'éclairage et de détection (2502, 2504) doit être ajustée.

30. Procédé selon l'une quelconque des revendications 21 à 29, comprenant par ailleurs le fait d'afficher une plage de pressions appliquées optimales ensemble avec la pression appliquée réelle appliquée par l'utilisateur.

31. Procédé selon l'une quelconque des revendications 21 à 29, comprenant par ailleurs le fait d'afficher une plage de pressions appliquées optimales qui correspond un état de pression transmurale nulle.

32. Procédé selon l'une quelconque des revendications 21 à 31, comprenant par ailleurs le fait de demander à l'utilisateur d'augmenter, de diminuer ou de maintenir la pression appliquée.

33. Procédé selon l'une quelconque des revendications 20 à 32, comprenant par ailleurs le fait d'afficher une sortie visuelle en temps réel du signal détecté et de la pression appliquée détectée.

34. Procédé selon l'une quelconque des revendications 22 à 33, dans lequel l'unité de rétroaction est un ordinateur portable comportant un processeur, une mémoire et un affichage.

35. Procédé selon l'une quelconque des revendications 22 à 34, dans lequel l'ensemble d'éclairage et de détection (2502, 2504), l'ensemble de détection de pression (2506, 2518) et l'unité de rétroaction (606) sont intégrés dans un dispositif portable.

36. Procédé selon la revendication 35, dans lequel le dispositif portable est configuré avec une pluralité d'ensembles d'éclairage et de détection et une pluralité d'ensembles de pression.

37. Procédé selon la revendication 34, comprenant par ailleurs le fait de communiquer sans fil entre l'unité de rétroaction (606) et l'ensemble d'éclairage et de détection (2502, 2504) et l'ensemble de détection de pression (2506, 2518).

38. Procédé selon la revendication 22, dans lequel le dispositif de traitement mobile personnel comprend l'unité de rétroaction.

39. Procédé selon l'une quelconque des revendications 21 à 38, dans lequel le signal détecté est un signal de photopléthysmographie, PPG.
